# EUROPEAN PATENT SPECIFICATION

(11) **EP 4 314 330 B1**
(45) Date of publication and mention of the grant of the patent: **14.10.2026**
(21) Application number: 22718118.7
(22) Date of filing: 24.03.2022
(51) Int. Cl.: C12Q 1/6806

(54) **HYBRIDIZATION BUFFER FORMULATIONS**
HYBRIDISIERUNGSPUFFERFORMULIERUNGEN
FORMULATIONS DE TAMPONS D'HYBRIDATION

(30) Priority: 26.03.2021 US 202163166324 P
(43) Date of publication of application: 07.02.2024
(73) Proprietor: F. Hoffmann-La Roche AG, 4070 Basel (CH); Roche Diagnostics GmbH, 68305 Mannheim (DE)
(72) Inventor: LEFKOWITZ, Joshua, Pleasanton, CA 94588 (US); NGUYEN, Duylinh, Pleasanton,CA 94588 (US); SOTO, Isabel, Pleasanton, CA 94588 (US); WADIA, Persis P., Pleasanton,CA 94588 (US)
(74) Representative: Göhring, Frank
(86) International application number: PCT/EP2022/057732
(87) International publication number: WO 2022/200485

(56) References cited:
- WO-A1-2015/165859
- WO-A1-2020/021084
- US-A1- 2002 187 476
- US-A1- 2003 190 660
- US-A1- 2005 136 413
- US-A1- 2020 102 611
- CULVER HEIDI R. ET AL: "Click Nucleic Acid-DNA Binding Behavior: Dependence on Length, Sequence, and Ionic Strength", BIOMACROMOLECULES, vol. 21, no. 10, 11 September 2020 (2020-09-11), US, pages 4205 - 4211, XP055944476, ISSN: 1525-7797, DOI: 10.1021/acs.biomac.0c00996

## Description

### BACKGROUND OF THE DISCLOSURE

Hybridization is a phenomenon in which single-stranded deoxyribonucleic acid (DNA) or ribonucleic acid (RNA) molecules anneal to complementary DNA or RNA. Though a double-stranded DNA sequence is generally stable under physiological conditions, changing these conditions in the laboratory (generally by raising the surrounding temperature) will cause the molecules to separate into single strands. These strands are complementary to each other but may also be complementary to other sequences present in their surroundings. Lowering the surrounding temperature allows the single-stranded molecules to anneal or "hybridize" to each other.

Hybridization is useful in numerous molecular biology techniques including Southern blots, Northern blots, primer extension, polymerase chain reaction (PCR), target enrichment, library preparation for next generation sequencing (NGS), and most approaches to DNA sequencing. Overall, genetic relatedness of two species can be determined by hybridizing segments of their DNA (DNA-DNA hybridization). A variety of different methods use hybridization to pinpoint the origin of a DNA sample, including polymerase chain reaction (PCR). In another technique, short DNA sequences are hybridized to cellular mRNAs to identify expressed genes. Researchers are also exploring the use of antisense RNA to bind to undesired mRNA, preventing the ribosome from translating the mRNA into protein.

Hybridization buffers are buffers in which hybridization might take place. Formamide is commonly used as a denaturant in hybridization buffers. While formamide is an effective denaturant, it is highly toxic (identified by the European Chemical Agency (ECHA) as a substance of very high concern' for its reproductive toxicity) and is hazardous to ship (the US Department of Transportation requires double-containment of formamide-containing solutions). It is desirable to prepare a hybridization buffer that maintains specificity and sensitivity, while avoiding toxic substances that are "Registration, Evaluation, Authorization and Restriction of Chemicals" (REACH) non-compliant. It is also desirable to prepare and use a hybridization buffer that is not susceptible to the formation of precipitates.

### BRIEF SUMMARY OF THE DISCLOSURE

Applicant has developed a hybridization buffer formulation that does not substantially interfere with hybridization chemistry, is capable of being utilized at temperatures below about 15°C without precipitation, and/or is REACH compliant.

Applicant has discovered that dimethyl sulfoxide (DMSO) may act as a substitute for formamide in hybridization buffers. Applicant has also discovered that the hybridization buffers disclosed herein which include DMSO are stable and do not form precipitates when stored at temperatures below about 10°C (e.g. below about 5°C, below about 0°C, below about -5°C, below about -10°C, below about - 15°C, below about -20°C) or when used in automated liquid handling instruments (e.g. the AVENIO Edge instrument available from Roche Molecular Systems, Inc., Pleasanton, CA).

A first aspect of the present disclosure is a hybridization buffer formulation comprising: (i) between about 36% to about 50% of DMSO by total volume of the hybridization buffer formulation; (ii) between about 0.0008% to about 0.0016% of one or more surfactants by total volume of the hybridization buffer formulation; (iii) between about 20% about 26% of one or more quaternary ammonium salts by total volume of the hybridization buffer formulation; (iv) between about 5% about 6.5% of one or more buffers by total volume of the hybridization buffer formulation; and (v) between 25% about 32% of one or more secondary salts by total volume of the hybridization buffer formulation. In some embodiments, the hybridization buffer formulation is substantially free from precipitates, e.g. precipitates derived from 2-(N-morpholino)ethanesulfonic acid. In some embodiments, the hybridization buffer formulation is free of formamide. In some embodiments, the hybridization buffer formulation is free from precipitates, e.g. precipitates derived from 2-(N-morpholino)ethanesulfonic acid.

In some embodiments, the one or more surfactants are anionic surfactants. In some embodiments, the one or more surfactants are selected from the group consisting of polysorbate 20, polysorbate 40, polysorbate 60, polysorbate 80, and combinations thereof. In some embodiments, the one or more surfactants is polysorbate 20.

In some embodiments, the one or more quaternary ammonium salts are selected from the group consisting of tetramethylammonium chloride, tetraethylammonium chloride, tetrabutylammonium chloride, trimethylammonium chloride, Tetra-n-butylammonium chloride, and combinations thereof. In some embodiments, the one or more quaternary ammonium salts is tetramethylammonium chloride.

In some embodiments, the one or more buffers are selected from the group consisting of 2-morpholinoethanesulfonic acid, 3-(N-morpholino)propanesulfonic acid, succinate, cacodylate, 4-(2-hydroxyethyl)-1-piperazineethanesulfonic acid, and combinations thereof. In some embodiments, the one or more buffers is 2-morpholinoethanesulfonic acid.

In some embodiments, the one or more surfactants comprises polysorbate 20, the one or more quaternary ammonium salts comprises tetramethylammonium chloride, the one or more buffers comprises 2-morpholinoethanesulfonic acid, and wherein the one or more secondary salts comprises N,N,N-trimethylglycine. In some embodiments, the hybridization buffer consists essentially of polysorbate 20, tetramethylammonium chloride, 2-morpholinoethanesulfonic acid, and N,N,N-trimethylglycine.

In some embodiments, the hybridization buffer formulation comprises: (i) between about 48% to about 52% of the DMSO by total volume of the hybridization buffer formulation; (ii) between about 0.0008% to about 0.0016% of the one or more surfactants by total volume of the hybridization buffer formulation; (iii) between about 18% about 22% of the one or more quaternary ammonium salts by total volume of the hybridization buffer formulation; (iv) between about 4% about 6% of the one or more buffers by total volume of the hybridization buffer formulation; and (v) between 24% about 26% of the one or more secondary salts by total volume of the hybridization buffer formulation. In some embodiments, the hybridization buffer comprises about 40% DMSO. In some embodiments, the one or more surfactants are anionic surfactants. In some embodiments, the one or more surfactants are selected from the group consisting of polysorbate 20, polysorbate 40, polysorbate 60, polysorbate 80, and combinations thereof. In some embodiments, the one or more surfactants is polysorbate 20. In some embodiments, the one or more quaternary ammonium salts are selected from the group consisting of tetramethylammonium chloride, tetraethylammonium chloride, tetrabutylammonium chloride, trimethylammonium chloride, Tetra-n-butylammonium chloride, and combinations thereof. In some embodiments, the one or more quaternary ammonium salts comprises tetramethylammonium chloride. In some embodiments, the one or more buffers are selected from the group consisting of 2-morpholinoethanesulfonic acid, 3-(N-morpholino)propanesulfonic acid, succinate, cacodylate, 4-(2-hydroxyethyl)-1-piperazineethanesulfonic acid, and combinations thereof. In some embodiments, the one or more buffers is 2-morpholinoethanesulfonic acid. In some embodiments, the one or more secondary salts are selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof. In some embodiments, the one or more secondary salts is N,N,N-trimethylglycine. In some embodiments, the one or more surfactants comprises polysorbate 20, the one or more quaternary ammonium salts is tetramethylammonium chloride, the one or more buffers comprises 2-morpholinoethanesulfonic acid, and wherein the one or more secondary salts comprises N,N,N-trimethylglycine. In some embodiments, the hybridization buffer consists essentially of polysorbate 20, tetramethylammonium chloride, 2-morpholinoethanesulfonic acid, and N,N,N-trimethylglycine.

In some embodiments, the hybridization buffer formulation comprises: (i) between about 38% to about 42% of the DMSO by total volume of the hybridization buffer formulation; (ii) between about 0.0008% to about 0.0016% of the one or more surfactants by total volume of the hybridization buffer formulation; (iii) between about 22% about 26% of the one or more quaternary ammonium salts by total volume of the hybridization buffer formulation; (iv) between about 5% about 7% of the one or more buffers by total volume of the hybridization buffer formulation; and (v) between 28% about 31% of the one or more secondary salts by total volume of the hybridization buffer formulation. In some embodiments, the hybridization buffer comprises about 50% DMSO. In some embodiments, the one or more surfactants are anionic surfactants. In some embodiments, the one or more surfactants are selected from the group consisting of polysorbate 20, polysorbate 40, polysorbate 60, polysorbate 80, and combinations thereof. In some embodiments, the one or more surfactants is polysorbate 20. In some embodiments, the one or more quaternary ammonium salts are selected from the group consisting of tetramethylammonium chloride, tetraethylammonium chloride, tetrabutylammonium chloride, trimethylammonium chloride, Tetra-n-butylammonium chloride, and combinations thereof. In some embodiments, the one or more quaternary ammonium salts is tetramethylammonium chloride. In some embodiments, the one or more buffers are selected from the group consisting of 2-morpholinoethanesulfonic acid, 3-(N-morpholino)propanesulfonic acid, succinate, cacodylate, 4-(2-hydroxyethyl)-1-piperazineethanesulfonic acid, and combinations thereof. In some embodiments, the one or more buffers comprises 2-morpholinoethanesulfonic acid. In some embodiments, the one or more secondary salts are selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof. In some embodiments, the one or more secondary salts is N,N,N-trimethylglycine. In some embodiments, the one or more surfactants comprises polysorbate 20, the one or more quaternary ammonium salts comprises tetramethylammonium chloride, the one or more buffers comprises 2-morpholinoethanesulfonic acid, and wherein the one or more secondary salts comprises N,N,N-trimethylglycine. In some embodiments, the hybridization buffer consists essentially of polysorbate 20, tetramethylammonium chloride, 2-morpholinoethanesulfonic acid, and N,N,N-trimethylglycine.

In some embodiments, the hybridization buffer formulation consists of: (i) between about 48% to about 52% of the DMSO by total volume of the hybridization buffer formulation; (ii) between about 0.0008% to about 0.0016% of the one or more surfactants by total volume of the hybridization buffer formulation; (iii) between about 18% about 22% of the one or more quaternary ammonium salts by total volume of the hybridization buffer formulation; (iv) between about 4% about 6% of the one or more buffers by total volume of the hybridization buffer formulation; and (v) between 24% about 26% of the one or more secondary salts by total volume of the hybridization buffer formulation. In some embodiments, the hybridization buffer comprises about 40% DMSO. In some embodiments, the one or more surfactants are anionic surfactants. In some embodiments, the one or more surfactants are selected from the group consisting of polysorbate 20, polysorbate 40, polysorbate 60, polysorbate 80, and combinations thereof. In some embodiments, the one or more surfactants is polysorbate 20. In some embodiments, the one or more quaternary ammonium salts are selected from the group consisting of tetramethylammonium chloride, tetraethylammonium chloride, tetrabutylammonium chloride, trimethylammonium chloride, Tetra-n-butylammonium chloride, and combinations thereof. In some embodiments, the one or more quaternary ammonium salts is tetramethylammonium chloride. In some embodiments, the one or more buffers are selected from the group consisting of 2-morpholinoethanesulfonic acid, 3-(N-morpholino)propanesulfonic acid, succinate, cacodylate, 4-(2-hydroxyethyl)-1-piperazineethanesulfonic acid, and combinations thereof. In some embodiments, the one or more buffers is 2-morpholinoethanesulfonic acid. In some embodiments, the one or more secondary salts are selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof. In some embodiments, the one or more secondary salts comprises N,N,N-trimethylglycine. In some embodiments, the one or more surfactants is polysorbate 20, the one or more quaternary ammonium salts comprises tetramethylammonium chloride, the one or more buffers comprises 2-morpholinoethanesulfonic acid, and wherein the one or more secondary salts comprises N,N,N-trimethylglycine. In some embodiments, the hybridization buffer consists essentially of polysorbate 20, tetramethylammonium chloride, 2-morpholinoethanesulfonic acid, and N,N,N-trimethylglycine.

A second aspect of the present disclosure is a reaction mixture comprising: (i) between about 18% to about 38% DMSO by total volume of the reaction mixture; (ii) between about 0.0008% to about 0.002% of one or more surfactants by total volume of the reaction mixture; (iii) between about 22% about 33% of one or more quaternary ammonium salts by total volume of the reaction mixture; (iv) between about 5% about 8% of one or more buffers by total volume of the reaction mixture; (v) between 28% about 42% of one or more secondary salts by total volume of the reaction mixture; and (vi) between about 0% to about 0.04% water. In some embodiments, the reaction mixture is free of formamide. In some embodiments, the reaction mixture is substantially free from precipitates, e.g. precipitates derived from 2-(N-morpholino)ethanesulfonic acid. In some embodiments, the reaction mixture is free from precipitates, e.g. precipitates derived from 2-(N-morpholino)ethanesulfonic acid.

In some embodiments, the one or more surfactants are anionic surfactants. In some embodiments, the one or more surfactants are selected from the group consisting of polysorbate 20, polysorbate 40, polysorbate 60, polysorbate 80, and combinations thereof. In some embodiments, the one or more surfactants is polysorbate 20.

In some embodiments, the one or more quaternary ammonium salts are selected from the group consisting of tetramethylammonium chloride, tetraethylammonium chloride, tetrabutylammonium chloride, trimethylammonium chloride, Tetra-n-butylammonium chloride, and combinations thereof. In some embodiments, the one or more quaternary ammonium salts comprises tetramethylammonium chloride.

In some embodiments, the one or more buffers are selected from the group consisting of 2-morpholinoethanesulfonic acid, 3-(N-morpholino)propanesulfonic acid, succinate, cacodylate, 4-(2-hydroxyethyl)-1-piperazineethanesulfonic acid, and combinations thereof. In some embodiments, the one or more buffers comprises 2-morpholinoethanesulfonic acid. The art taught that 2-morpholinoethanesulfonic acid was not very soluble in DMSO (see, for example, Taha and Coutinho, "Organic-phase biological buffers for biochemical and biological research in organic media," Journal of Molecular Liquids 221:197-205 (2016)). Therefore, the art taught away from a buffer containing both 2-morpholinoethanesulfonic acid and DMSO.

In some embodiments, the one or more secondary salts are selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof. In some embodiments, the one or more secondary salts comprises N,N,N-trimethylglycine.

In some embodiments, the one or more surfactants is polysorbate 20, the one or more quaternary ammonium salts comprises tetramethylammonium chloride, the one or more buffers is 2-morpholinoethanesulfonic acid, and wherein the one or more secondary salts is N,N,N-trimethylglycine. In some embodiments, the hybridization buffer consists essentially of polysorbate 20, tetramethylammonium chloride, 2-morpholinoethanesulfonic acid, N,N,N-trimethylglycine, and water.

In some embodiments, the reaction mixture comprises (i) between about 21% to about 36% of the DMSO by total volume of the reaction mixture; (ii) between about 0.0008% to about 0.002% of the one or more surfactants by total volume of the reaction mixture; (iii) between about 24% about 32% of the one or more quaternary ammonium salts by total volume of the reaction mixture; (iv) between about 5% about 8% of the one or more buffers by total volume of the reaction mixture; (v) between 28% about 42% of the one or more secondary salts by total volume of the reaction mixture; and (vi) between about 0% to about 0.04% of the water. In some embodiments, the one or more surfactants is polysorbate 20, the one or more quaternary ammonium salts comprises tetramethylammonium chloride, the one or more buffers comprises 2-morpholinoethanesulfonic acid, and wherein the one or more secondary salts comprises N,N,N-trimethylglycine. In some embodiments, the hybridization buffer consists essentially of polysorbate 20, tetramethylammonium chloride, 2-morpholinoethanesulfonic acid, N,N,N-trimethylglycine, and water.

In some embodiments, the reaction mixture consists essentially of (i) between about 21% to about 36% of the DMSO by total volume of the reaction mixture; (ii) between about 0.0008% to about 0.002% of the one or more surfactants by total volume of the reaction mixture; (iii) between about 24% about 32% of the one or more quaternary ammonium salts by total volume of the reaction mixture; (iv) between about 5% about 8% of the one or more buffers by total volume of the reaction mixture; (v) between 28% about 42% of the one or more secondary salts by total volume of the reaction mixture; and (vi) between about 0% to about 0.04% of the water. In some embodiments, the one or more surfactants comprises polysorbate 20, the one or more quaternary ammonium salts comprises tetramethylammonium chloride, the one or more buffers comprises 2-morpholinoethanesulfonic acid, and wherein the one or more secondary salts comprises N,N,N-trimethylglycine. In some embodiments, the hybridization buffer consists essentially of polysorbate 20, tetramethylammonium chloride, 2-morpholinoethanesulfonic acid, N,N,N-trimethylglycine, and water.

In some embodiments, the reaction mixture consists of (i) between about 21% to about 36% of the DMSO by total volume of the reaction mixture; (ii) between about 0.0008% to about 0.002% of the one or more surfactants by total volume of the reaction mixture; (iii) between about 24% about 32% of the one or more quaternary ammonium salts by total volume of the reaction mixture; (iv) between about 5% about 8% of the one or more buffers by total volume of the reaction mixture; (v) between 28% about 42% of the one or more secondary salts by total volume of the reaction mixture; and (vi) between about 0% to about 0.04% of the water. In some embodiments, the one or more surfactants is polysorbate 20, the one or more quaternary ammonium salts comprises tetramethylammonium chloride, the one or more buffers comprises 2-morpholinoethanesulfonic acid, and wherein the one or more secondary salts comprises N,N,N-trimethylglycine. In some embodiments, the hybridization buffer consists essentially of polysorbate 20, tetramethylammonium chloride, 2-morpholinoethanesulfonic acid, N,N,N-trimethylglycine, and water.

A third aspect of the present disclosure is a master mix comprising: (a) between about 65% to about 75% of a reaction mixture by total weight of the master mix, wherein the reaction mixtures comprises: i) between about 21% to about 36% DMSO by total volume of the reaction mixture; (ii) between about 0.0008% to about 0.002% of one or more surfactants by total volume of the reaction mixture; (iii) between about 24% about 32% of one or more quaternary ammonium salts by total volume of the reaction mixture; (iv) between about 5% about 8% of one or more buffers by total volume of the reaction mixture; (v) between 28% about 42% of one or more secondary salts by total volume of the reaction mixture; and (vi) between about 0% to about 0.04% water; and (b) between about 25% to about 35% of oligonucleotides by total volume of the master mix. In some embodiments, the master mix comprises between about 67% to about 71% of the reaction mixture. In some embodiments, the master mix comprises about 70% of the reaction mixture. In some embodiments, the master mix is free of formamide. In some embodiments, the reaction mixture is substantially free from precipitates, e.g. precipitates derived from 2-(N-morpholino)ethanesulfonic acid. In some embodiments, the master mix is free from precipitates, e.g. precipitates derived from 2-(N-morpholino)ethanesulfonic acid.

A fourth aspect of the present disclosure is a master mix consisting essentially of: (a) between about 65% to about 75% of a reaction mixture by total volume of the master mix, wherein the reaction mixture comprises i) between about 21% to about 36% DMSO by total volume of the reaction mixture; (ii) between about 0.0008% to about 0.002% of one or more surfactants by total volume of the reaction mixture; (iii) between about 24% about 32% of one or more quaternary ammonium salts by total volume of the reaction mixture; (iv) between about 5% about 8% of one or more buffers by total volume of the reaction mixture; (v) between 28% about 42% of one or more secondary salts by total volume of the reaction mixture; and (vi) between about 0% to about 0.04% water; and (b) between about 25% to about 35% of oligonucleotides by total volume of the master mix. In some embodiments, the master mix comprises between about 67% to about 71% of the reaction mixture. In some embodiments, the master mix comprises about 70% of the reaction mixture.

A fifth aspect of the present disclosure is a master mix consisting of: (a) between about 65% to about 75% of a reaction mixture by total weight of the master mix, wherein the reaction mixture comprises: i) between about 21% to about 36% DMSO by total volume of the reaction mixture; (ii) between about 0.0008% to about 0.002% of one or more surfactants by total volume of the reaction mixture; (iii) between about 24% about 32% of one or more quaternary ammonium salts by total volume of the reaction mixture; (iv) between about 5% about 8% of one or more buffers by total volume of the reaction mixture; (v) between 28% about 42% of one or more secondary salts by total volume of the reaction mixture; and (vi) between about 0% to about 0.04% water; and (b) between about 25% to about 35% of oligonucleotides by total weight of the master mix. In some embodiments, the master mix comprises between about 67% to about 71% of the reaction mixture. In some embodiments, the master mix comprises about 70% of the reaction mixture.

### BRIEF DESCRIPTION OF THE FIGURES

For a general understanding of the features of the disclosure, reference is made to the drawings. In the drawings, like reference numerals have been used throughout to identify identical elements. The patent or application file contains at least one drawing execute din color. Copies of this paten tor patent application publication with color drawing(s) will be provided by the Office upon request and payment of the necessary fee.

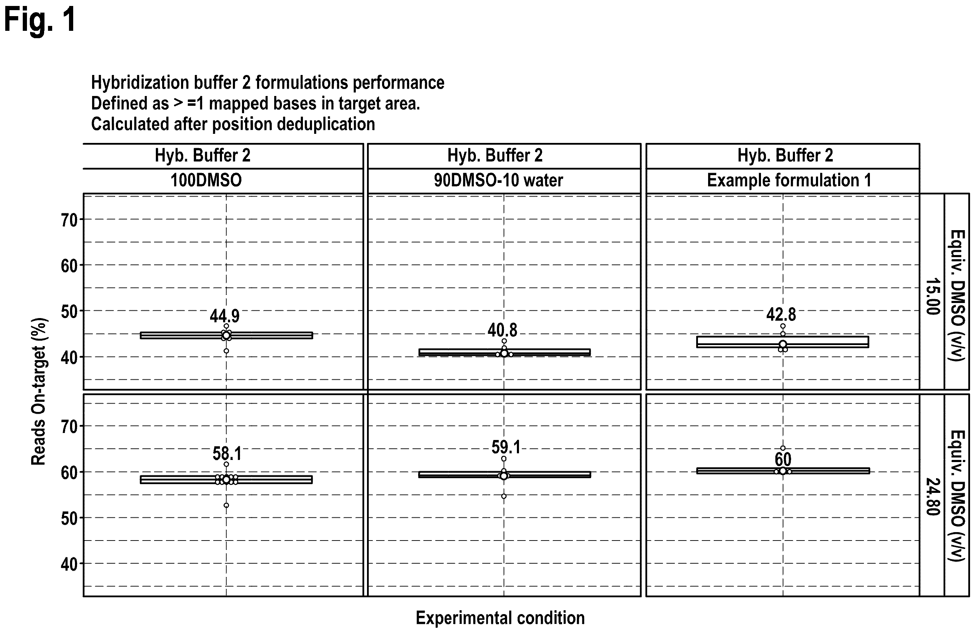

FIG. 1 illustrates the percentage of reads on-target. Specifically, FIG. 1 depicts the percent of position-deduplicated reads over all mapped reads at a sample level. For each experimental condition a boxplot was generated. The lower and upper hinges denote the 25th and the 75th percentile, respectively, with the median denoted with a blue doth with the y-value displayed (this is equal to the central line in the boxplot); whiskers are calculated as 1.5*IQR (IQR, Inter-Quartile Range). The grid columns denote the hybridization buffer 2 formulation. Left to right: Pure DMSO (100% v/v %), 90%DMSO-10%water (v/v %), Example formulation 1 (50%DMSO v/v %). The grid rows show the effective amount of pure DMSO in the reaction as a percent of the hybridization master mix as a percent (%).

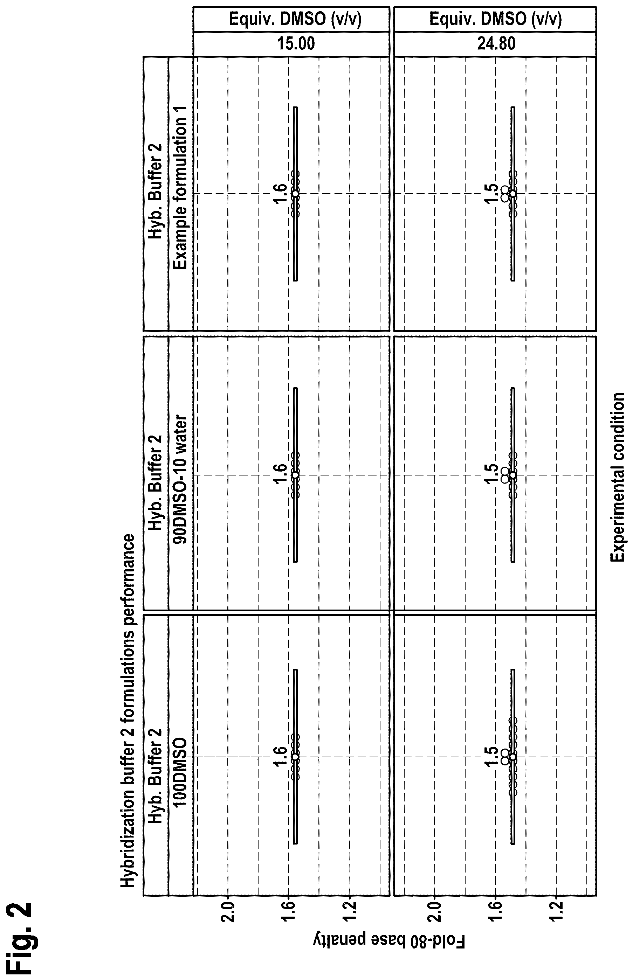

FIG. 2 illustrates the fold-80 base penalty. Specifically, FIG. 2 depicts the fold over-coverage necessary to raise 80% of bases in with non-zero coverage to the mean coverage level in those targets (developed by the Broad Institute). For each experimental condition a boxplot was generated for each distribution. The lower and upper hinges denote the 25th and the 75 percentile, respectively, with the median denoted with a blue doth with the y-value (this is also the central line in the boxplot); whiskers are calculated as 1.5*IQR (IQR=inter-quartile range). The grid columns denote the hybridization buffer 2 formulation. From left to right: Pure DMSO (100% v/v %), 90%DMSO-10%water (v/v %), and Formulation Example 1 (50%DMSO v/v %). The grid rows show the effective amount of pure DMSO in the reaction as a percent of the hybridization master mix as a percent (%).

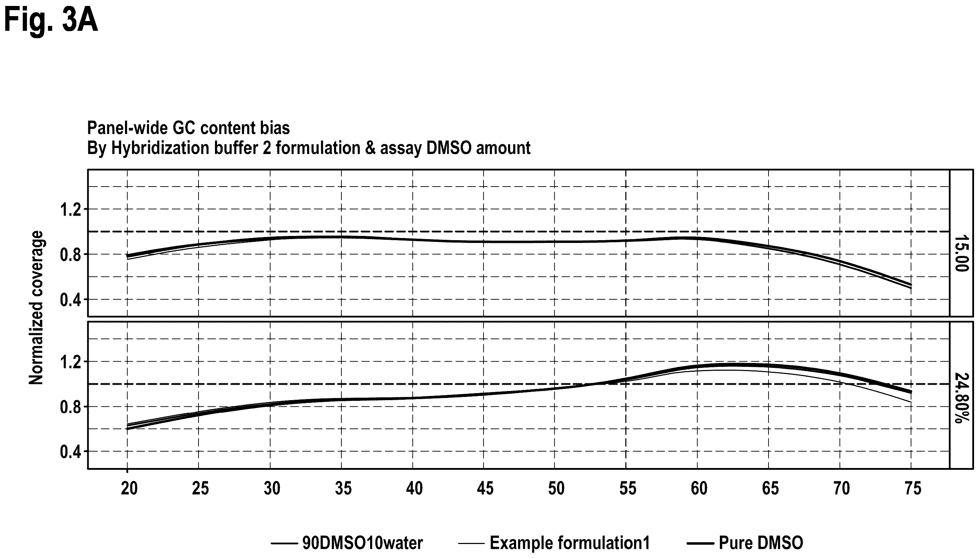

FIG. 3A illustrates the GC-content bias. Specifically, FIG. 3A depicts the line of best fit for normalized GC-coverage for each condition in five percent bins. Normalized coverage is the ratio of coverage in a given bin with respect to the sample-wide average coverage. Trend-line colors denote the hybridization buffer 2 formulation (red = Pure DMSO (100% v/v %), Blue = Formulation Example 1 (50%DMSO v/v %), Green =90%DMSO-10%water (v/v %)). The top graph is subdivided by the effective amount of pure DMSO in the reaction as a percent of the hybridization master mix (15 and 24.8%).

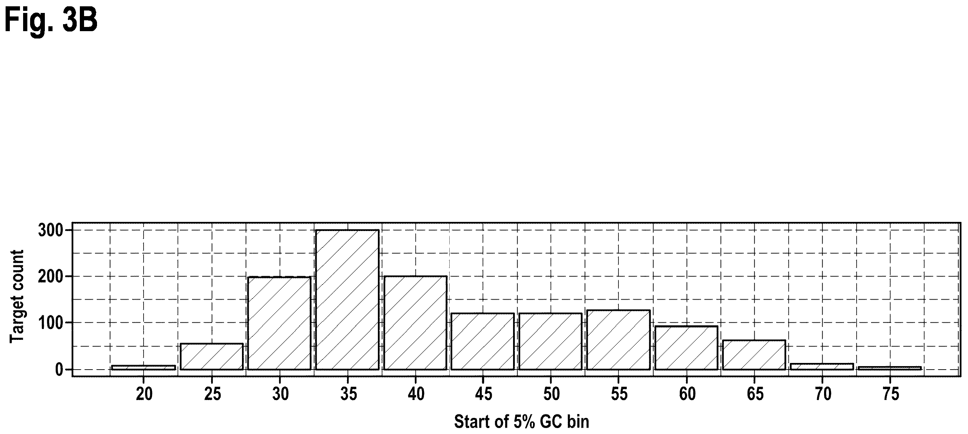

FIG. 3B reports the total number of targets in each of the same five percent bins used in FIG. 3A.

### DETAILED DESCRIPTION

It should also be understood that, unless clearly indicated to the contrary, in any methods claimed herein that include more than one step or act, the order of the steps or acts of the method is not necessarily limited to the order in which the steps or acts of the method are recited.

### Definitions

As used herein, the singular terms "a," "an," and "the" include plural referents unless context clearly indicates otherwise. Similarly, the word "or" is intended to include "and" unless the context clearly indicates otherwise. The term "includes" is defined inclusively, such that "includes A or B" means including A, B, or A and B.

As used herein in the specification and in the claims, "or" should be understood to have the same meaning as "and/or" as defined above. For example, when separating items in a list, "or" or "and/or" shall be interpreted as being inclusive, e.g., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (e.g. "one or the other but not both") when preceded by terms of exclusivity, such as "either," "one of," "only one of" or "exactly one of." "Consisting essentially of," when used in the claims, shall have its ordinary meaning as used in the field of patent law.

The terms "comprising," "including," "having," and the like are used interchangeably and have the same meaning. Similarly, "comprises," "includes," "has," and the like are used interchangeably and have the same meaning. Specifically, each of the terms is defined consistent with the common United States patent law definition of "comprising" and is therefore interpreted to be an open term meaning "at least the following," and is also interpreted not to exclude additional features, limitations, aspects, etc. Thus, for example, "a device having components a, b, and c" means that the device includes at least components a, b, and c. Similarly, the phrase: "a method involving steps a, b, and c" means that the method includes at least steps a, b, and c. Moreover, while the steps and processes may be outlined herein in a particular order, the skilled artisan will recognize that the ordering steps and processes may vary.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

As used herein, the terms "amplification" or "amplify" refer to a process in which a copy number increases. Amplification may be a process in which replication occurs repeatedly over time to form multiple copies of a template. Amplification can produce an exponential or linear increase in the number of copies as amplification proceeds. Exemplary amplification strategies include polymerase chain reaction (PCR), loop-mediated isothermal amplification (LAMP), rolling circle replication (RCA), cascade-RCA, nucleic acid based amplification (NASBA), and the like. Also, amplification can utilize a linear or circular template. Amplification can be performed under any suitable temperature conditions, such as with thermal cycling or isothermally. Furthermore, amplification can be performed in an amplification mixture (or reagent mixture), which is any formulation capable of amplifying a nucleic acid target, if any, in the mixture. PCR amplification relies on repeated cycles of heating and cooling (i.e., thermal cycling) to achieve successive rounds of replication. PCR can be performed by thermal cycling between two or more temperature setpoints, such as a higher denaturation temperature and a lower annealing/extension temperature, or among three or more temperature setpoints, such as a higher denaturation temperature, a lower annealing temperature, and an intermediate extension temperature, among others. PCR can be performed with a thermostable polymerase, such as Taq DNA polymerase. PCR generally produces an exponential increase in the amount of a product amplicon over successive cycles.

As used herein, the term "complementary" generally refers to the capability for precise pairing between two nucleotides. The term "complementary" refers to the ability to form favorable thermodynamic stability and specific pairing between the bases of two nucleotides at an appropriate temperature and ionic buffer conditions. Complementarity is achieved by distinct interactions between the nucleobases adenine, thymine (uracil in RNA), guanine and cytosine, where adenine pairs with thymine or uracil, and guanine pairs with cytosine. For example, if a nucleotide at a given position of a nucleic acid is capable of hydrogen bonding with a nucleotide of another nucleic acid, then the two nucleic acids are considered to be complementary to one another at that position. Complementarity between two single-stranded nucleic acid molecules may be "partial," in which only some of the nucleotides bind, or it may be complete when total complementarity exists between the single-stranded molecules. A first nucleotide sequence can be said to be the "complement" of a second sequence if the first nucleotide sequence is complementary to the second nucleotide sequence. A first nucleotide sequence can be said to be the "reverse complement" of a second sequence, if the first nucleotide sequence is complementary to a sequence that is the reverse (i.e., the order of the nucleotides is reversed) of the second sequence.

As used herein, the terms "nucleic acid" or "polynucleotide" (used interchangeably herein) refer to a polymeric form of nucleotides of any length, either deoxyribonucleotides or ribonucleotides, or analogs thereof. Polynucleotides may have any three-dimensional structure, and may perform any function, known or unknown. Unless specifically limited, the terms encompass nucleic acids or polynucleotides including known analogues of natural nucleotides that have similar binding properties as the reference nucleic acid and are metabolized in a manner similar to naturally occurring nucleotides. Non-limiting examples of polynucleotides include coding or non-coding regions of a gene or gene fragment, loci (locus) defined from linkage analysis, exons, introns, messenger RNA (mRNA), transfer RNA, ribosomal RNA, ribozymes, cDNA, synthetic polynucleotides, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified, such as by conjugation with a labeling component. Unless otherwise indicated, a particular nucleic acid sequence also implicitly encompasses conservatively modified variants thereof (e.g., degenerate codon substitutions), alleles, orthologues, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

As used herein, the term "oligonucleotide," refers to an oligomer of nucleotide or nucleoside monomer units wherein the oligomer optionally includes non-nucleotide monomer units, and/or other chemical groups attached at internal and/or external positions of the oligomer. The oligomer can be natural or synthetic and can include naturally-occurring oligonucleotides, or oligomers that include nucleosides with non-naturally-occurring (or modified) bases, sugar moieties, phosphodiester-analog linkages, and/or alternative monomer unit chiralities and isomeric structures (e.g., 5'- to 2'-linkage, L-nucleosides, α-anomer nucleosides, β-anomer nucleosides, locked nucleic acids (LNA), peptide nucleic acids (PNA)).

As used herein, the term "substantially" means the qualitative condition of exhibiting total or near-total extent or degree of a characteristic or property of interest. In some embodiments, "substantially" means within about 2.5%. In some embodiments, "substantially" means within about 5%. In some embodiments, "substantially" means within about 7.5%. In some embodiments, "substantially" means within about 10%. In some embodiments, "substantially" means within about 12.5%. In some embodiments, "substantially" means within about 15%. In some embodiments, "substantially" means within about 20%.

### OVERVIEW

The present disclosure is directed to hybridization buffers, reaction mixtures, and master mixes suitable for enrichment of DNA oligonucleotides. As described herein, Applicant has discovered that the disclosed hybridization buffer formulations do not interfere with hybridization chemistry, are capable of being utilized at temperatures below about 15°C without precipitation, and/or are REACH compliant. Applicants have also discovered that the hybridization buffers disclosed herein which include DMSO are stable and do not form precipitates when stored at temperatures at or below about 15°C , at or below at or about 10°C, at or below about 5°C , at or below about 0°C, at or below about -5°C, at or below about -10°C, at or below - about 15°C, at or below about -20°C, etc.

### HYBRIDIZATION BUFFER FORMULATIONS

In some embodiments, the hybridization buffer formulations of the present disclosure include dimethyl sulfoxide (DMSO), one or more quaternary ammonium salts, one or more secondary salts, one or more buffers, and one or more surfactants. In other embodiments, the hybridization buffer formulations of the present disclosure include dimethyl sulfoxide (DMSO), one or more quaternary ammonium salts, one or more secondary salts, one or more buffers, one or more surfactants, and water. In yet other embodiments, the hybridization buffer formulations of the present disclosure consist essentially of dimethyl sulfoxide (DMSO), one or more quaternary ammonium salts, one or more secondary salts, one or more buffers, one or more surfactants, and water. In yet other embodiments, the hybridization buffer formulations of the present disclosure consist of dimethyl sulfoxide (DMSO), one or more quaternary ammonium salts, one or more secondary salts, one or more buffers, one or more surfactants, and water.

In some embodiments, the hybridization buffer formulations are able to be stored for between about 3 to about 18 months at a temperature ranging from between about -20°C to about -15°C without the formation of substantially any precipitates. In other embodiments, the hybridization buffer formulations are able to be stored for between about 3 to about 12 months at a temperature ranging from between about -20°C to about -15°C without the formation of substantially any precipitates. In yet other embodiments, the hybridization buffer formulations are able to be stored for between about 3 to about 6 months at a temperature ranging from between about -20°C to about -15°C without the formation of substantially any precipitates.

In some embodiments, the hybridization buffer formulations of the present disclosure are capable of being stored at temperatures below about 18°C without precipitation of 2-(N-morpholino)ethanesulfonic acid. In some embodiments, the hybridization buffer formulations of the present disclosure are capable of being stored at temperatures below about 15°C without precipitation of 2-(N-morpholino)ethanesulfonic acid. In some embodiments, the hybridization buffer formulations of the present disclosure are capable of being stored at temperatures below about 10°C without precipitation of 2-(N-morpholino)ethanesulfonic acid. In some embodiments, the hybridization buffer formulations of the present disclosure are capable of being stored at temperatures below about 5°C without precipitation of 2-(N-morpholino)ethanesulfonic acid. In some embodiments, the hybridization buffer formulations of the present disclosure are capable of being stored at temperatures below about 0°C without precipitation of 2-(N-morpholino)ethanesulfonic acid. In some embodiments, the hybridization buffer formulations of the present disclosure are capable of being stored at temperatures below about -5°C without precipitation of 2-(N-morpholino)ethanesulfonic acid. In some embodiments, the hybridization buffer formulations of the present disclosure are capable of being stored at temperatures below about -10°C without precipitation of 2-(N-morpholino)ethanesulfonic acid. In some embodiments, the hybridization buffer formulations of the present disclosure are capable of being stored at temperatures below about -15°C without precipitation of 2-(N-morpholino)ethanesulfonic acid. In some embodiments, the hybridization buffer formulations of the present disclosure are capable of being stored at temperatures below about -20°C without precipitation of 2-(N-morpholino)ethanesulfonic acid. In some embodiments, the hybridization buffer formulations of the present disclosure are capable of being stored at temperatures below -25°C without precipitation of 2-(N-morpholino)ethanesulfonic acid.

### Dimethyl Sulfoxide (DMSO)

In some embodiments, the hybridization buffer formulation includes DMSO. DMSO is an organosulfur compound with the formula (CH₃)₂SO. This colorless liquid is a polar aprotic solvent that dissolves both polar and nonpolar compounds and is miscible in a wide range of organic solvents as well as water. In some embodiments, DMSO is used in polymerase chain reaction (PCR) to inhibit secondary structures in the DNA template or the DNA primers.

Alternatively, in some embodiments, DMSO may also be utilized as a denaturant in nucleic acid (e.g. DNA, RNA) target enrichment, which is a process by which nucleic acid sequences (e.g. DNA) may be bound to and isolated using custom probes (e.g. oligonucleotides designed to bind to one or more target sequences within a sample including one or more nucleic acid sequences). In some embodiments, for effective binding, probes must have the right conditions to be able to bind to the target, and conditions must be stringent enough to keep any non-target or semi-complimentary sequences from binding in place of the probes. In some embodiments, these conditions are created through warm temperatures and through the use of chemical denaturants. In some embodiments, DMSO allows the sequence specificity needed to bind at a given temperature to be increased. In combination with warm temperatures, in some embodiments, the length and approximate sequence specificity may be engineered such that nucleic acid sequences may hybridize to one another and/or to one or more probes. Examples of systems and method of target enrichment are disclosed in US Publication Nos. 2018/0016630, 2020/0048694, 2017/0037459, and 2018/0087108

In some embodiments, the hybridization buffer formulation comprises between about 25% to about 60% DMSO by total volume of the hybridization buffer formulation. In some embodiments, the hybridization buffer formulation comprises between about 28% to about 59% DMSO by total volume of the hybridization buffer formulation. In some embodiments, the hybridization buffer formulation comprises between about 28% to about 58% DMSO by total volume of the hybridization buffer formulation. In some embodiments, the hybridization buffer formulation comprises between about 28% to about 57% DMSO by total volume of the hybridization buffer formulation. In some embodiments, the hybridization buffer formulation comprises between about 28% to about 56% DMSO by total volume of the hybridization buffer formulation. In some embodiments, the hybridization buffer formulation comprises between about 29% to about 55% DMSO by total volume of the hybridization buffer formulation. In some embodiments, the hybridization buffer formulation comprises between about 30% to about 55% DMSO by total volume of the hybridization buffer formulation. In some embodiments, the hybridization buffer formulation comprises between about 31% to about 55% DMSO by total volume of the hybridization buffer formulation. In some embodiments, the hybridization buffer formulation comprises between about 32% to about 54% DMSO by total volume of the hybridization buffer formulation. In some embodiments, the hybridization buffer formulation comprises between about 33% to about 53% DMSO by total volume of the hybridization buffer formulation. In some embodiments, the hybridization buffer formulation comprises between about 33% to about 52% DMSO by total volume of the hybridization buffer formulation. In some embodiments, the hybridization buffer formulation comprises between about 34% to about 51% DMSO by total volume of the hybridization buffer formulation. In some embodiments, the hybridization buffer formulation comprises between about 33% to about 52% DMSO by total volume of the hybridization buffer formulation. In some embodiments, the hybridization buffer formulation comprises between about 35% to about 50% DMSO by total volume of the hybridization buffer formulation. In some embodiments, the hybridization buffer formulation comprises between about 36% to about 50% DMSO by total volume of the hybridization buffer formulation.

In some embodiments, the hybridization buffer formulation comprises between about 35% DMSO by total volume of the hybridization buffer formulation. In some embodiments, the hybridization buffer formulation comprises between about 37% DMSO by total volume of the hybridization buffer formulation. In some embodiments, the hybridization buffer formulation comprises between about 38% DMSO by total volume of the hybridization buffer formulation. In some embodiments, the hybridization buffer formulation comprises between about 39% DMSO by total volume of the hybridization buffer formulation. In some embodiments, the hybridization buffer formulation comprises between about 40% DMSO by total volume of the hybridization buffer formulation. In some embodiments, the hybridization buffer formulation comprises between about 41% DMSO by total volume of the hybridization buffer formulation. In some embodiments, the hybridization buffer formulation comprises between about 43% DMSO by total volume of the hybridization buffer formulation. In some embodiments, the hybridization buffer formulation comprises between about 43% DMSO by total volume of the hybridization buffer formulation.

In some embodiments, the hybridization buffer formulation comprises between about 45% DMSO by total volume of the hybridization buffer formulation. In some embodiments, the hybridization buffer formulation comprises between about 47% DMSO by total volume of the hybridization buffer formulation. In some embodiments, the hybridization buffer formulation comprises between about 48% DMSO by total volume of the hybridization buffer formulation. In some embodiments, the hybridization buffer formulation comprises between about 49% DMSO by total volume of the hybridization buffer formulation. In some embodiments, the hybridization buffer formulation comprises between about 50% DMSO by total volume of the hybridization buffer formulation. In some embodiments, the hybridization buffer formulation comprises between about 51% DMSO by total volume of the hybridization buffer formulation. In some embodiments, the hybridization buffer formulation comprises between about 52% DMSO by total volume of the hybridization buffer formulation. In some embodiments, the hybridization buffer formulation comprises between about 54% DMSO by total volume of the hybridization buffer formulation. In some embodiments, the hybridization buffer formulation comprises between about 55% DMSO by total volume of the hybridization buffer formulation.

### Surfactants

In some embodiments, the hybridization buffer formulation comprises between about 0.0001% to about 0.02% of one or more surfactants by total volume of the hybridization buffer formulation. In some embodiments, the hybridization buffer formulation comprises between about 0.0008% to about 0.002% of one or more surfactants by total volume of the hybridization buffer formulation. In some embodiments, the hybridization buffer formulation comprises between about 0.0008% to about 0.002% of one or more surfactants by total volume of the hybridization buffer formulation. In some embodiments, the hybridization buffer formulation comprises between about 0.0008% to about 0.0017% of one or more surfactants by total volume of the hybridization buffer formulation. In some embodiments, the hybridization buffer formulation comprises between about 0.0008% to about 0.0016% of one or more surfactants by total volume of the hybridization buffer formulation. In some embodiments, the hybridization buffer formulation comprises between about 0.0009% to about 0.0015% of one or more surfactants by total volume of the hybridization buffer formulation.

In some embodiments, the hybridization buffer formulation comprises between about 0.0009% of one or more surfactants by total volume of the hybridization buffer formulation. In some embodiments, the hybridization buffer formulation comprises between about 0.001% of one or more surfactants by total volume of the hybridization buffer formulation. In some embodiments, the hybridization buffer formulation comprises between about 0.0012% of one or more surfactants by total volume of the hybridization buffer formulation. In some embodiments, the hybridization buffer formulation comprises between about 0.00125% of one or more surfactants by total volume of the hybridization buffer formulation. In some embodiments, the hybridization buffer formulation comprises between about 0.0013% of one or more surfactants by total volume of the hybridization buffer formulation. In some embodiments, the hybridization buffer formulation comprises between about 0.0015% of one or more surfactants by total volume of the hybridization buffer formulation. In some embodiments, the hybridization buffer formulation comprises between about 0.0015% of one or more surfactants by total volume of the hybridization buffer formulation.

In some embodiments, the hybridization buffer formulation comprises between about 0.0009% of one or more anionic surfactants by total volume of the hybridization buffer formulation. In some embodiments, the hybridization buffer formulation comprises between about 0.001% of one or more anionic surfactants by total volume of the hybridization buffer formulation. In some embodiments, the hybridization buffer formulation comprises between about 0.0012% of one or more anionic surfactants by total volume of the hybridization buffer formulation. In some embodiments, the hybridization buffer formulation comprises between about 0.00125% of one or more anionic surfactants by total volume of the hybridization buffer formulation. In some embodiments, the hybridization buffer formulation comprises between about 0.0013% of one or more anionic surfactants by total volume of the hybridization buffer formulation. In some embodiments, the hybridization buffer formulation comprises between about 0.0015% of one or more anionic surfactants by total volume of the hybridization buffer formulation. In some embodiments, the hybridization buffer formulation comprises between about 0.0015% of one or more anionic surfactants by total volume of the hybridization buffer formulation.

In some embodiments, the surfactant is an anionic surfactant. Anionic surfactants are generally based upon sulfates, sulfonates, phosphates, or carboxylates and contain a water-soluble cation. A representative formula of a sulfonate is R-SO₃-M, where R is a hydrocarbon group of from about 5 to 22 carbon atoms which may be linked through an alkoxy or oxyalkoxy to the sulfonate functionality, and where M is a water-soluble cation such as an alkali metal. In some embodiments, anionic surfactants include alkyl ether sulfates, alkyl sulfates and sulfonates, alkyl carboxylates, alkyl phenyl ether sulfates, sodium salts of alkyl poly(oxyethylene) sulfonates, sodium salts of alkyl benzyl sulfonate, such as sodium salts of dodecylbenzyl sulfonate and sodium lauryl ether sulfate. In some embodiments, anionic surfactants also include anionic phosphate esters.

In some embodiments, the anionic surfactants include, but are not limited to polyoxyethylene alkyl ether, wherein the alkyl is (CH₂)_{S} and the oxyethylene is (C₂H₄O)_{T}, wherein S is an integer from 5 to 16, from 8 to 14, or from 10 to 12; and T is an integer from 10 to 40, from 15 to 30, or from 20 to 28. In one embodiment, the anionic surfactant is polyoxyethylene lauryl ether having a formula (C₂H₄O)₂₃C₁₂H₂₅OH. In another embodiment, the anionic surfactant is a polyoxyethylene (20) sorbitan monoalkylate, the monoalkylate comprising between 8 and 14 carbons. In another embodiment, the anionic surfactant is a linear secondary alcohol polyoxyethylene having a formula C₁₂-₁₄H₂₅-₂₉0(CH₂CH₂O]ₓ, wherein x is an integer ranging from between 2 and 12. In yet another embodiment, the anionic surfactant is a polyoxyethylene octyl phenyl ether. Exemplary surfactants are sold under the names: Brij^{®} 35 (e.g. Polyoxyethylene lauryl ether), TWEEN^{®}, Tergitol^{™}, Triton^{™} (e.g. Polyethylene glycol p-(1,1,3,3-tetramethylbutyl)-phenyl ether), Ecosurf^{™}, Dowfax^{™}, polysorbate 20 (Tween 20) polysorbate 80^{™} (e.g. Polyethylene glycol sorbitan monolaurate), BigCHAP (e.g. 3-{Dimethyl[3-(4-{5,9,16-trihydroxy-2,15-dimethyltetracyclo[8.7.0.02,7.011,15]), Deoxy BigCHAP (e.g. N,N-bis-(3-D-Gluconamidopropyl)deoxycholamide), IGEPAL^{®} (e.g. octylphenoxypolyethoxyethanol), Saponin (e.g. triterpene glycosides), Thesit^{®} (e.g. Hydroxypolyethoxydodecane), Nonidet^{®} (e.g. octylphenoxypolyethoxyethanol), Pluronic F-68 (e.g. Polyoxyethylene-polyoxypropylene block copolymer), digitonin, deoxycholate (e.g. 3α,12α-Dihydroxy-5β-cholanic acid sodium salt), and the like. In some embodiments, the anionic surfactant is selected from Brij^{®} 35, TWEEN^{®}, Tergitol^{™}, Triton^{™}.

In some embodiments, the surfactant is a cationic surfactant. Cationic surfactants useful in formulations of the present disclosure include amino or quaternary ammonium moieties. Cationic surfactants among those useful herein are disclosed in the following documents: M.C. Publishing Co., McCutcheon's, Detergents & Emulsifiers, (North American edition 1979); Schwartz, et al.; Surface Active Agents, Their Chemistry and Technology, New York: Interscience Publishers, 1949; U.S. Pat. No. 3,155,591, Hilfer, issued Nov. 3, 1964; U.S. Pat. No. 3,929,678, Laughlin et al., issued Dec. 30, 1975; U.S. Pat. No. 3,959,461, Bailey et al., issued May 25, 1976; and U.S. Pat. No. 4,387,090, Bolich, Jr., issued Jun. 7, 1983.

In some embodiments, the quaternary ammonium-containing cationic surfactant materials useful herein are those of the general formula: 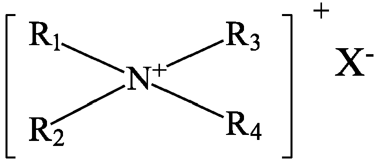 wherein R₁-R₄ are each independently an aliphatic group of from about 1 to about 22 carbon atoms or an aromatic, alkoxy, polyoxyalkylene, alkylamido, hydroxyalkyl, aryl or alkylaryl group having from about 1 to about 22 carbon atoms; and X is a salt-forming anion such as those selected from halogen, (e.g. chloride, bromide), acetate, citrate, lactate, glycolate, phosphate nitrate, sulfate, and alkylsulfate radicals. In some embodiments, the aliphatic groups may include, in addition to carbon and hydrogen atoms, ether linkages, and other groups such as amino groups. In some embodiments, the longer chain aliphatic groups, e.g., those of about 12 carbons, or higher, can be saturated or unsaturated. In some embodiments, the cationic surfacants are mono-long chain (e.g., mono C₁₂ to C₂₂, or C₁₂ to C₁₈) di-short chain (e.g., C₁ to C₃ alkyl) quaternary ammonium salts.

In some embodiments, the salts of primary, secondary and tertiary fatty amines are also suitable cationic surfactant materials. In some embodiments, the alkyl groups of such amines have from about 12 to about 22 carbon atoms and may be substituted or unsubstituted. Such amines include, but are not limited to, stearamido propyl dimethyl amine, diethyl amino ethyl stearamide, dimethyl stearamine, dimethyl soyamine, soyamine, myristyl amine, tridecyl amine, ethyl stearylamine, N-tallowpropane diamine, ethoxylated (with 5 moles of ethylene oxide) stearylamine, dihydroxy ethyl stearylamine, and arachidylbehenylamine. Suitable amine salts include the halogen, acetate, phosphate, nitrate, citrate, lactate, and alkyl sulfate salts. Such salts include, but are not limited to, stearylamine hydrochloride, soyamine chloride, stearylamine formate, N-tallowpropane diamine dichloride, stearamidopropyl dimethylamine citrate, cetyl trimethyl ammonium chloride and dicetyl diammonium chloride. In some embodiments, the cationic surfactant is a cetyl trimethyl ammonium chloride, stearyl trimethyl ammonium chloride, tetradecyltrimethly ammonium chloride, dicetyldimethyl ammonium chloride, dicocodimethyl ammonium chloride and mixtures thereof. In other embodiments, the cationic surfactant is a cetyl trimethyl ammonium chloride.

In some embodiments, the surfactant is a non-ionic surfactant. Among the suitable nonionic surfactants are condensation products of C₈-C₃₀ alcohols with sugar or starch polymers. These compounds can be represented by the formula (S)ₙ -O-R, wherein S is a sugar moiety such as glucose, fructose, mannose, and galactose; n is an integer of from about 1 to about 1000, and R is C₈-C₃₀ alkyl. Examples of suitable C₈-C₃₀ alcohols from which the R group may be derived include decyl alcohol, cetyl alcohol, stearyl alcohol, lauryl alcohol, myristyl alcohol, oleyl alcohol, and the like. Suitable examples of such surfactants include decyl polyglucoside and lauryl polyglucoside.

Other suitable nonionic surfactants include the condensation products of alkylene oxides with fatty acids (i.e., alkylene oxide esters of fatty acids). These materials have the general formula RCO(X)ₙ OH, wherein R is a C₁₀-C₃₀ alkyl, X is -OCH₂CH₂- (derived from ethylene oxide) or -OCH₂CHCH₃- (derived from propylene oxide), where n is an integer from about 1 to about 200.

Yet other suitable nonionic surfactants are the condensation products of alkylene oxides with fatty acids (i.e., alkylene oxide diesters of fatty acids) having the formula RCO(X)ₙOOCR, wherein R is a C₁₀-C₃₀ alkyl, X is -OCH₂CH₂-(derived from ethylene oxide) or -OCH₂CHCH₃- (derived from propylene oxide), where n is an integer from about 1 to about 200. Yet other nonionic surfactants are the condensation products of alkylene oxides with fatty alcohols (i.e., alkylene oxide ethers of fatty alcohols) having the general formula R(X)ₙOR', wherein R is C₁₀-C₃₀ alkyl, where n is an integer from about 1 to about 200, and R' is H or a C₁₀-C₃₀ alkyl.

Yet further nonionic surfactants are compounds having the formula RCO(X)ₙOR' where R and R' are C₁₀-C₃₀ alkyl, X is -OCH₂CH₂- (derived from ethylene oxide) or -OCH₂CHCH₃- (derived from propylene oxide), and n is an integer from about 1 to about 200. Examples of alkylene oxide-derived nonionic surfactants include ceteth-1, ceteth-2, ceteth-6, ceteth-10, ceteth-12, ceteraeth-2, ceteareth6, ceteareth-10, ceteareth-12, steareth-1, steareth-2, stearteth-6, steareth-10, steareth-12, PEG-2 stearate, PEG4 stearate, PEG6 stearate, PEG-10 stearate, PEG-12 stearate, PEG-20 glyceryl stearate, PEG-80 glyceryl tallowate, PPG-10 glyceryl stearate, PEG-30 glyceryl cocoate, PEG-80 glyceryl cocoate, PEG-200 glyceryl tallowate, PEG-8 dilaurate, PEG-10 distearate, and mixtures thereof. Still other useful nonionic surfactants include polyhydroxy fatty acid amides disclosed, for example, in U.S. Pat. Nos. 2,965,576, 2,703,798, and 1,985,424.

Non-limiting examples of surfactants include Tomadol 1200 (Air Products), Tomadol 900 (Air Products), Tomadol 91-8 (Air Products), Tomadol 1-9 (Air Products), Tergitol 15-S-9 (Sigma), Tergitol 15-S-12 (Sigma), Masurf NRW-N (Pilot Chemical), Bio-Soft N91-6 (Stepan), and Brij-35 (Polyethylene glycol dodecyl ether) (Sigma).

In some embodiments, the surfactant is selected from Polyhydroxyethyl alkoxy alkylene oxides, Polyoxyethylene-polyoxyprolyene block co-polymers, Etherified polyoxyethylene-polyoxyprolyene block co-polymers, Modified alkylated polyols, Modified/Methyl capped block co-polymers, Non-Ionic polyols, Non-ionic surfactants, Alkoxylated polyols., Alkyl polyglycosides, Glucoethers, Alkoxylated alcohols, Alcohol ethoxylates, Polyoxytheylene, Anioinic blends, Ethylene oxides, Nonylphenol ethoxylates, Sodium laureth sulfates, Laureth sulfates, Ammonium laureth sulfates, TEA lauryl sulfate, Diethylhexyl sodium sulfosuccinate, Sodium lauroyl sarcosinates, Sodium stearate, Sodium olefin sulfonate, Disodium laureth sulfosuccinate, Disodium oleamine sulfosuccinate, Sodium dioctyl sulfosuccinate Sodium cocoyl isethionate, Sodium capryloyl isethionate, Sodium caproyl isethionate, Sodium lauroyl isethionate, Sodium palmitoyl isethionate, Acrylates/Steareth-20 itaconate copolymer, Ammonium capryleth sulfate, Ammonium pareth-25 sulfate, Ammonium myreth sulfate, Ceteareth-20, Cocamidopropyl betaines, Disteareth-75 IPDI, -100 IPDI, Emulsifying wax NF, Isosteareth-20, Steareth - 2, -4, 10, 16, -20, 21, Isosteareth -2, -10, -20, Magnesium laureth sulfate, Magnesium oleth sulfate, Polyethylene glycols, PEG-20, PEG-40, Phenoxyethanol, olyoxyethylene, Polysorbate-20, -40, -60, -80, Steareth-2, -4, -10, -16, -20, -21, Sodium coceth sulfate, Sodium deceth sulfate, Sodium oleth sulfate, Sodium laureth sulfate, Sodium syreth sulfate, Sodium trideceth sulfate, Zinc coceth sulfate, 2-Dodecylbenzenesulfonic acid, 4-Dodecylbenzenesulfonic acid, Alkylbenzene sulfonates, Glucoheptonates, odium glucoheptonate, Potassium glucoheptonate, Calcium glucoheptonate, Magnesium glucoheptonate, Boron glucoheptonate, Chlorine glucoheptonate, Copper glucoheptonate, Iron glucoheptonate, Manganese glucoheptonate, Molybdenum glucoheptonate, Zinc glucoheptonate, Methanoic acid, Ethanoic acid, Propanoic acid, Butanoic acid, Pentanoic acid, Hexanoic acid, Heptanoic acid, Octanoic acid, Nonanoic acid, Decanoic acid, Undecanoic acid, Dodecanoic acid, Tridecanoic acid, Tetradecanoic acid, Pentadecanoic acid, Hexadecanoic acid, Heptadecanoic acid, Octadecanoic acid, Nonadecanoic acid, Icosanoic acid, 1,2,3-trihydroxypropane, Diethylene glycol, Alkylphenol ethoxylate, 3-oxapentane-1,5-diol, Propane-1,2,3-triol, Alkylphenol ethoxylate, Polydimethylsiloxane,1,2-Propanediol, Dimethylpolysiloxane, Fatty alcohol and butoxyethanol, Butoxyethanol, Phosphate ester surfactant, Alkyl aryl alkoxylate, Hydroxy carboxylic acids, Citric acid, Tartaric acid, Gluconic acid, Oxalic acid, Propionic acids, Phosphate ester, Ammonium sulfates, Ethoxylated surfactants, Sodium hydroxide, Anticorrosion compounds, Sequestering agents, Nonionic and Ionic surfactants, Hydroxy carboxylates, Polyacrylates, Sugar acrylates, Aminocarboxylic acid base, Phosphate(s), Phosphonate(s), Sodium hexameta phosphate, sodium polyphosphate, Sodium tripolyphosphate, Sodium trimeta phosphate, Sodium pyrophosphates, Phosphonated aminopolycarboxylates, Amino polycarboxylates, EDTMP, DETMP, ATMP, HEDP, DTPMP, Polyether-polymethylsiloxane copolymer(s), Ethoxylated alkyl phosphate esters, C16-C28 alkanoates, Paraffin base petroleum oil, Agricultural paraffinic oil, Alkanolamide surfactants, Alkylaryl polyethoxyethanol sulfates, Alkylaryl polyoxyethylene glycol phosphate ester surfactants, Phosphate ester surfactants, Petroleum oil, Polyol fatty acid ester, Methylated seed oil, Paraffinic oil, Carbonyldiamide polyoxyalkylated glycol adduct, Carbonyl diamine, Polyoxyethylene-polyoxypropylene polymer, Methylated vegetable oil, Corn-derived surfactants, Free fatty acids, Isoproponal, Alkyl aryl polyoxyethylene glycols, Hydrogen sulfate, Aliphatic hydrocarbon oils, Polyacrylic acid salts, Polysiloxane polyether copolymer, Polyalkyleneoxide modified polydimethylsiloxane, Tall oil fatty acids, Organosilicone surfactant, Polyalkylene modified heptamethyltrisiloxane, Modified alkanoates, Poly fatty acid esters, Carbonate salts, Polysiloxane, Limonene, Allyloxypolyethyleneglycol methyl ether, Phytobland base oil, Dimethylpolysiloxane, Mineral oil, Polyether polymethylsiloxane copolymer, Nonionic carbohydrate surfactants, Polyoxythylenepolyoxyethylene-polyoxypropylene glycol, Monocarbamide dihydrogen sulfate, Antifoaming agents, Crop-based elasto polymer, Diammonium salts, and any combination thereof.

In some embodiments, the surfactant is a polyhydroxyethyl alkoxy alkylene oxide. In some embodiments, the surfactant is a polyoxyethylene-polyoxyprolyene block copolymer. In some embodiments, the surfactant is an etherified polyoxyethylene-polyoxyprolyene block copolymer. In some embodiments, the surfactant is a modified alkylated polyol. In some embodiments, the surfactant is a modified/methyl capped block copolymer. In some embodiments, the surfactant is a non-ionic polyol. In some embodiments, the surfactant is an alkoxylated polyol. In some embodiments, the surfactant is an alkyl polyglycoside. In some embodiments, the surfactant is a glucoether. In some embodiments, the surfactant is an alkoxylated alcohol. In some embodiments, the surfactant is an alcohol ethoxylate. In some embodiments, the surfactant is a polyoxytheylene. In some embodiments, the surfactant is an anionic blend.

In some embodiments, the surfactant is polysorbate 20. In other embodiments, the surfactant is polysorbate 40. In yet other embodiments, the surfactant is polysorbate 60. In further embodiments, the surfactant is polysorbate 80. In some embodiments, the hybridization buffer formulation comprises between about 0.0009% of one of polysorbate 20, polysorbate 40, polysorbate 60, or polysorbate 80 by total volume of the hybridization buffer formulation. In some embodiments, the hybridization buffer formulation comprises between about 0.001% of one of polysorbate 20, polysorbate 40, polysorbate 60, or polysorbate 80 by total volume of the hybridization buffer formulation. In some embodiments, the hybridization buffer formulation comprises between about 0.0012% of one of polysorbate 20, polysorbate 40, polysorbate 60, or polysorbate 80 by total volume of the hybridization buffer formulation. In some embodiments, the hybridization buffer formulation comprises between about 0.00125% of one of polysorbate 20, polysorbate 40, polysorbate 60, or polysorbate 80 by total volume of the hybridization buffer formulation. In some embodiments, the hybridization buffer formulation comprises between about 0.0013% of one of polysorbate 20, polysorbate 40, polysorbate 60, or polysorbate 80 by total volume of the hybridization buffer formulation. In some embodiments, the hybridization buffer formulation comprises between about 0.0015% of one of polysorbate 20, polysorbate 40, polysorbate 60, or polysorbate 80 by total volume of the hybridization buffer formulation. In some embodiments, the hybridization buffer formulation comprises between about 0.0015% of one of polysorbate 20, polysorbate 40, polysorbate 60, or polysorbate 80 by total volume of the hybridization buffer formulation.

### Quaternary Ammonium Salts

**In** some embodiments, the hybridization buffer formulation comprises between about 15% about 33% of one or more quaternary ammonium salts by total volume of the hybridization buffer formulation. In some embodiments, the hybridization buffer formulation comprises between about 15% about 32% of one or more quaternary ammonium salts by total volume of the hybridization buffer formulation. In some embodiments, the hybridization buffer formulation comprises between about 16% about 31% of one or more quaternary ammonium salts by total volume of the hybridization buffer formulation. In some embodiments, the hybridization buffer formulation comprises between about 17% about 30% of one or more quaternary ammonium salts by total volume of the hybridization buffer formulation. In some embodiments, the hybridization buffer formulation comprises between about 17% about 29% of one or more quaternary ammonium salts by total volume of the hybridization buffer formulation. In some embodiments, the hybridization buffer formulation comprises between about 18% about 28% of one or more quaternary ammonium salts by total volume of the hybridization buffer formulation. In some embodiments, the hybridization buffer formulation comprises between about 19% about 27% of one or more quaternary ammonium salts by total volume of the hybridization buffer formulation. In some embodiments, the hybridization buffer formulation comprises between about 20% about 26% of one or more quaternary ammonium salts by total volume of the hybridization buffer formulation.

In some embodiments, the hybridization buffer formulation comprises about 16% of one or more quaternary ammonium salts by total volume of the hybridization buffer formulation. In some embodiments, the hybridization buffer formulation comprises about 18% of one or more quaternary ammonium salts by total volume of the hybridization buffer formulation. In some embodiments, the hybridization buffer formulation comprises about 19% of one or more quaternary ammonium salts by total volume of the hybridization buffer formulation. In some embodiments, the hybridization buffer formulation comprises about 20% of one or more quaternary ammonium salts by total volume of the hybridization buffer formulation. In some embodiments, the hybridization buffer formulation comprises about 21% of one or more quaternary ammonium salts by total volume of the hybridization buffer formulation. In some embodiments, the hybridization buffer formulation comprises about 22% of one or more quaternary ammonium salts by total volume of the hybridization buffer formulation. In some embodiments, the hybridization buffer formulation comprises about 23% of one or more quaternary ammonium salts by total volume of the hybridization buffer formulation. In some embodiments, the hybridization buffer formulation comprises about 24% of one or more quaternary ammonium salts by total volume of the hybridization buffer formulation. In some embodiments, the hybridization buffer formulation comprises about 25% of one or more quaternary ammonium salts by total volume of the hybridization buffer formulation. In some embodiments, the hybridization buffer formulation comprises about 26% of one or more quaternary ammonium salts by total volume of the hybridization buffer formulation. In some embodiments, the hybridization buffer formulation comprises about 28% of one or more quaternary ammonium salts by total volume of the hybridization buffer formulation. In some embodiments, the hybridization buffer formulation comprises about 30% of one or more quaternary ammonium salts by total volume of the hybridization buffer formulation.

As used herein, the term "quaternary ammonium salt" refers to a tetravalent nitrogen-containing molecule with a positive charge on nitrogen and a counter ion. Such quaternary ammonium salts include aliphatic and aromatic substituents. Examples of aliphatic quaternary ammonium salts include tetra alkyl ammonium chloride, such as tetramethyl ammonium chloride, tetraethyl ammonium chloride, etc. Examples of aromatic quaternary ammonium salts include benzalkonium chloride, benzethonium chloride, etc.

In some embodiments, the quaternary ammonium salt is a compound having the formula:

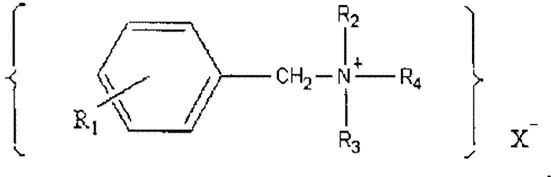

wherein R₁ is selected from the group consisting of H and C₁-C₁₂ straight or branched chain alkyl; R₂ and R₃ are each independently -CH₃, -CH₂OH, and -CH₂CH₂OH; R₄ is (a) -CH₃, (b) C₂ - C₂₂ straight or branched chain alkyl, (c) C₂-C₂₂ straight or branched chain alkenyl, or (d) [CH₂CH₂O-]ₙ-R₅, where n is an integer ranging from 1 to 3, and R₅ is H, C₁-C₁₂ straight or branched chain alkyl, C₂ - C₂₂ straight or branched alkenyl; or a moiety having the formula: 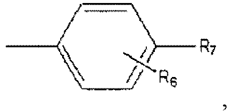 where R₆ is selected from H and -CH₃, and R₇ is selected from of C₁ - C₂₂ straight or branched chain alkyl, and C₂ - C₂₂ straight or branched chain alkenyl, and (e) -(CH₂)ₘNOCR₇ or -(CH₂)ₘCONR, where m is an integer ranging from 1 - 3, R₇ is as described above; and X is a pharmaceutically acceptable counterion.

In some embodiments, the quaternary ammonium salt may be benzalkonium chloride; benzalkonium saccharinate; behenalkonium chloride; cetalkonium chloride; erucalkonium chloride; lauralkonium chloride; myristalkonium chloride; myristalkonium saccharinate (Quaternium-3); stearalkonium chloride; olealkonium chloride; tallowalkonium chloride; dodecylbenzyttrimethylammonium chloride (Quaternium-28); dodecylbenzyl trimethyl ammonium 2-ethylhexanoate; ethylbenzyl alkyldimethylammonium cyclohexylsulfanamate (Quaternium-8); ethylbenzyl dimethyl dodecyl ammonium chloride (Quaternium-14); dodecylbenzyl dimethyl octadecyl ammonium chloride; dodecylbenzyl triethanol ammonium chloride (Quaternium-30); benzoxonium chloride; benzylbis (2-hydroxyethyl) (2-dodecyloxyethyl) ammonium bromide; benzylbis (2-hydroxyethyl) (2-dodecyloxyethyl) ammonium chloride; benzethonium chloride; methylbenzethonium chloride; N, N- (diethyl-N- [2- [4- ( 1, 1,3,3-tetramethylbutyl) phenoxy] ethyl] benzenemethanaminium chloride (phenoctide); dodecarbonium chloride; babassuamidopropalkonium chloride; and wheatgermamidopropalkonium chloride.

In some embodiments, the quaternary ammonium is benzalkonium chloride, stearalkonium, behenalkonium chloride, olealkonium chloride, erucalkonium chloride, benzethonium chloride, methylbenzethonium chloride, phenoctide, wheatgermamidopropalkonium chloride and babassuamidopropalkonium chloride, or a mixture thereof. In some embodiments, the quaternary ammonium salt enhancer is benzethonium chloride. In a further aspect of the invention, the quaternary ammonium salt is methylbenzethonium chloride. In some embodiments, the quaternary ammonium salt is benzalkonium chloride. In some embodiments, the quaternary ammonium salt is olealkonium chloride. In some embodiments, the quaternary ammonium salt is phenoctide.

In some embodiments, the quaternary ammonium salt is a member selected from the group consisting of alkyl-, dimethyl benzenemethanaminium salts; acyl-, dimethyl benzenemethanaminium salts; mixed acyl-/alkyl-, dimethyl benzenemethanaminium salts; ethylbenzyl dodecyl dimethylammonium chloride, dodecylbenzyltrimethylammonium chloride, dodecylbenzyl triethanolammonium chloride, benzoxonium chloride, benzethonium chloride; methylbenzethonium chloride; phenoctide; dodecarbonium chloride; and mixed alkyl-/acyl-, amidopropalkonium salts, or a mixture thereof.

In some embodiments, the quaternary ammonium salt is benzethonium chloride, benzalkonium chloride, methylbenzethonium chloride, cetalkonium chloride, cetylpyridinium chloride, cetrimonium, cetrimide, dofanium chloride, tetraethylammonium bromide, didecyldimethylammonium chloride or domiphen bromide. In some embodiments, the azole is an imidazole, such as bifonazole, butoconazole, clotrimazole, econazole, fenticonazole, isoconazole, ketoconazole, miconazole, omoconazole, oxiconazole, sertaconazole, sulconazole or tioconazole. In other embodiments, the azole is a triazole, such as albaconazole, fluconazole, isavuconazole, itraconazol, posaconazole, ravuconazole, terconazole or voriconazole. In yet other embodiments, the azole is a thiazole, such as abafungin. In some embodiments, the quaternary ammonium salt is benzethonium chloride and the azole is fluconazole. Formulations including combinations of two or more of the quaternary ammonium salts with two or more azoles are also within the scope of the present disclosure.

In some embodiments, the quaternary ammonium salt is tetramethylammonium chloride. In some embodiments, the quaternary ammonium salt is tetramethylammonium bromide. In some embodiments, the quaternary ammonium salt is tetramethylammonium iodide.

In some embodiments, the quaternary ammonium salt is tetraethylammonium chloride. In some embodiments, the quaternary ammonium salt is tetraethylammonium bromide. In some embodiments, the quaternary ammonium salt is tetraethylammonium iodide.

In some embodiments, the quaternary ammonium salt is tetrabutylammonium chloride. In some embodiments, the quaternary ammonium salt is tetrabutylammonium bromide. In some embodiments, the quaternary ammonium salt is tetrabutylammonium iodide.

In some embodiments, the quaternary ammonium salt is Tetra-n-butylammonium chloride. In some embodiments, the quaternary ammonium salt is Tetra-n-butylammonium bromide. In some embodiments, the quaternary ammonium salt is Tetra-n-butylammonium iodide.

In some embodiments, the quaternary ammonium salt is trimethylammonium chloride. In some embodiments, the quaternary ammonium salt is trimethylammonium bromide. In some embodiments, the quaternary ammonium salt is trimethylammonium iodide.

In some embodiments, the hybridization buffer formulation comprises about 18% of one or more quaternary ammonium salts selected from the group consisting of tetramethylammonium chloride, tetraethylammonium chloride, tetrabutylammonium chloride, trimethylammonium chloride, Tetra-n-butylammonium chloride, and combinations thereof by total volume of the hybridization buffer formulation. In some embodiments, the hybridization buffer formulation comprises about 19% of one or more quaternary ammonium salts selected from the group consisting of tetramethylammonium chloride, tetraethylammonium chloride, tetrabutylammonium chloride, trimethylammonium chloride, Tetra-n-butylammonium chloride, and combinations thereof by total volume of the hybridization buffer formulation. In some embodiments, the hybridization buffer formulation comprises about 20% of one or more quaternary ammonium salts selected from the group consisting of tetramethylammonium chloride, tetraethylammonium chloride, tetrabutylammonium chloride, trimethylammonium chloride, Tetra-n-butylammonium chloride, and combinations thereof by total volume of the hybridization buffer formulation. In some embodiments, the hybridization buffer formulation comprises about 21% of one or more quaternary ammonium salts selected from the group consisting of tetramethylammonium chloride, tetraethylammonium chloride, tetrabutylammonium chloride, trimethylammonium chloride, Tetra-n-butylammonium chloride, and combinations thereof by total volume of the hybridization buffer formulation. In some embodiments, the hybridization buffer formulation comprises about 22% of one or more quaternary ammonium salts selected from the group consisting of tetramethylammonium chloride, tetraethylammonium chloride, tetrabutylammonium chloride, trimethylammonium chloride, Tetra-n-butylammonium chloride, and combinations thereof by total volume of the hybridization buffer formulation. In some embodiments, the hybridization buffer formulation comprises about 23% of one or more quaternary ammonium salts selected from the group consisting of tetramethylammonium chloride, tetraethylammonium chloride, tetrabutylammonium chloride, trimethylammonium chloride, Tetra-n-butylammonium chloride, and combinations thereof by total volume of the hybridization buffer formulation. In some embodiments, the hybridization buffer formulation comprises about 24% of one or more quaternary ammonium salts selected from the group consisting of tetramethylammonium chloride, tetraethylammonium chloride, tetrabutylammonium chloride, trimethylammonium chloride, Tetra-n-butylammonium chloride, and combinations thereof by total volume of the hybridization buffer formulation. In some embodiments, the hybridization buffer formulation comprises about 25% of one or more quaternary ammonium salts selected from the group consisting of tetramethylammonium chloride, tetraethylammonium chloride, tetrabutylammonium chloride, trimethylammonium chloride, Tetra-n-butylammonium chloride, and combinations thereof by total volume of the hybridization buffer formulation. In some embodiments, the hybridization buffer formulation comprises about 26% of one or more quaternary ammonium salts selected from the group consisting of tetramethylammonium chloride, tetraethylammonium chloride, tetrabutylammonium chloride, trimethylammonium chloride, Tetra-n-butylammonium chloride, and combinations thereof by total volume of the hybridization buffer formulation. In some embodiments, the hybridization buffer formulation comprises about 28% of one or more quaternary ammonium salts selected from the group consisting of tetramethylammonium chloride, tetraethylammonium chloride, tetrabutylammonium chloride, trimethylammonium chloride, Tetra-n-butylammonium chloride, and combinations thereof by total volume of the hybridization buffer formulation.

### Buffers

In some embodiments, the hybridization buffer formulation comprises between about 2% about 10% of one or more buffers by total volume of the hybridization buffer formulation. In some embodiments, the hybridization buffer formulation comprises between about 2% about 8% of one or more buffers by total volume of the hybridization buffer formulation. In some embodiments, the hybridization buffer formulation comprises between about 3% about 7% of one or more buffers by total volume of the hybridization buffer formulation. In some embodiments, the hybridization buffer formulation comprises between about 4% about 7% of one or more buffers by total volume of the hybridization buffer formulation. In some embodiments, the hybridization buffer formulation comprises between about 5% about 7% of one or more buffers by total volume of the hybridization buffer formulation.

In some embodiments, the hybridization buffer formulation comprises about 4% of one or more buffers by total volume of the hybridization buffer formulation. In some embodiments, the hybridization buffer formulation comprises about 4.5% of one or more buffers by total volume of the hybridization buffer formulation. In some embodiments, the hybridization buffer formulation comprises about 5% of one or more buffers by total volume of the hybridization buffer formulation. In some embodiments, the hybridization buffer formulation comprises about 5.5% of one or more buffers by total volume of the hybridization buffer formulation. In some embodiments, the hybridization buffer formulation comprises about 6% of one or more buffers by total volume of the hybridization buffer formulation. In some embodiments, the hybridization buffer formulation comprises about 6.5% of one or more buffers by total volume of the hybridization buffer formulation. In some embodiments, the hybridization buffer formulation comprises about 7% of one or more buffers by total volume of the hybridization buffer formulation.

Non-liming examples of buffers include citric acid, potassium dihydrogen phosphate, boric acid, diethyl barbituric acid, piperazine-N,N'-bis(2-ethanesulfonic acid), dimethylarsinic acid, 2-(N-morpholino)ethanesulfonic acid, tris(hydroxymethyl)methylamine (TRIS), 2-(N-morpholino)ethanesulfonic acid (TAPS), N,N-bis(2-hydroxyethyl)glycine(Bicine), N-tris(hydroxymethyl)methylglycine (Tricine), 4-2-hydroxyethyl-1-piperazineethanesulfonic acid (HEPES), 2-{[tris(hydroxymethyl)methyl]amino}ethanesulfonic acid (TES), and combinations thereof. In other embodiments, the buffer may be comprised of tris(hydroxymethyl)methylamine (TRIS), 2-(N-morpholino)ethanesulfonic acid (TAPS), N,N-bis(2-hydroxyethyl)glycine(Bicine), N - tris(hydroxymethyl)methylglycine (Tricine), 4-2-hydroxyethyl-1-piperazineethanesulfonic acid (HEPES), 2-{[tris(hydroxymethyl)methyl]amino}ethanesulfonic acid (TES), or a combination thereof.

In some embodiments, the buffer is 2-morpholinoethanesulfonic acid. In some embodiments, the buffer is 3-(N-morpholino)propanesulfonic acid ("MOPS"). In some embodiments, the buffer is succinate (PK2). In some embodiments, the surfactant is maleate (PK2). In some embodiments, the buffer is cacodylate. In some embodiments, the buffer is HEPES (4-(2-hydroxyethyl)-1-piperazineethanesulfonic acid).

**In** some embodiments, the hybridization buffer formulation comprises about 4% of 2-morpholinoethanesulfonic acid by total volume of the hybridization buffer formulation. In some embodiments, the hybridization buffer formulation comprises about 4.5% of 2-morpholinoethanesulfonic acid by total volume of the hybridization buffer formulation. In some embodiments, the hybridization buffer formulation comprises about 5% of 2-morpholinoethanesulfonic acid by total volume of the hybridization buffer formulation. In some embodiments, the hybridization buffer formulation comprises about 5.5% of 2-morpholinoethanesulfonic acid by total volume of the hybridization buffer formulation. In some embodiments, the hybridization buffer formulation comprises about 6% of 2-morpholinoethanesulfonic acid by total volume of the hybridization buffer formulation. In some embodiments, the hybridization buffer formulation comprises about 6.5% of 2-morpholinoethanesulfonic acid by total volume of the hybridization buffer formulation. In some embodiments, the hybridization buffer formulation comprises about 7% of 2-morpholinoethanesulfonic acid by total volume of the hybridization buffer formulation.

### Secondary Salts

**In** some embodiments, the hybridization buffer formulation comprises between 20% about 35% of one or more secondary salts by total volume of the hybridization buffer formulation. In some embodiments, the hybridization buffer formulation comprises between 21% about 35% of one or more secondary salts by total volume of the hybridization buffer formulation. In some embodiments, the hybridization buffer formulation comprises between 21% about 34% of one or more secondary salts by total volume of the hybridization buffer formulation. In some embodiments, the hybridization buffer formulation comprises between 22% about 33% of one or more secondary salts by total volume of the hybridization buffer formulation. In some embodiments, the hybridization buffer formulation comprises between 23% about 32% of one or more secondary salts by total volume of the hybridization buffer formulation. n some embodiments, the hybridization buffer formulation comprises between 24% about 32% of one or more secondary salts by total volume of the hybridization buffer formulation. n some embodiments, the hybridization buffer formulation comprises between 25% about 32% of one or more secondary salts by total volume of the hybridization buffer formulation.

In some embodiments, the hybridization buffer formulation comprises about 23% of one or more secondary salts by total volume of the hybridization buffer formulation. In some embodiments, the hybridization buffer formulation comprises about 24% of one or more secondary salts by total volume of the hybridization buffer formulation. In some embodiments, the hybridization buffer formulation comprises about 24.5% of one or more secondary salts by total volume of the hybridization buffer formulation. In some embodiments, the hybridization buffer formulation comprises about 25% of one or more secondary salts by total volume of the hybridization buffer formulation. In some embodiments, the hybridization buffer formulation comprises about 26% of one or more secondary salts by total volume of the hybridization buffer formulation. In some embodiments, the hybridization buffer formulation comprises about 28% of one or more secondary salts by total volume of the hybridization buffer formulation. In some embodiments, the hybridization buffer formulation comprises about 28.5% of one or more secondary salts by total volume of the hybridization buffer formulation. In some embodiments, the hybridization buffer formulation comprises about 29% of one or more secondary salts by total volume of the hybridization buffer formulation. In some embodiments, the hybridization buffer formulation comprises about 29.5% of one or more secondary salts by total volume of the hybridization buffer formulation. In some embodiments, the hybridization buffer formulation comprises about 30% of one or more secondary salts by total volume of the hybridization buffer formulation. In some embodiments, the hybridization buffer formulation comprises about 30.5% of one or more secondary salts by total volume of the hybridization buffer formulation. In some embodiments, the hybridization buffer formulation comprises about 31% of one or more secondary salts by total volume of the hybridization buffer formulation.

In some embodiments, the secondary salt of the amino acid is N,N,N-trimethylalanine. In some embodiments, the secondary salt is N,N,N-trimethylvaline. In some embodiments, the secondary salt of the amino acid is N,N,N-trimethylglycine (betaine). In some embodiments, the secondary salt of the amino acid is N,N,N-trimethylisolucine. In some embodiments, the secondary salt of the amino acid is N,N,N-trimethylmethionine.

In some embodiments, the secondary salt is tetramethylammonium chloride. In some embodiments, the secondary salt is tetramethylammonium bromide. In some embodiments, the secondary salt is tetramethylammonium iodide.

In some embodiments, the hybridization buffer formulation comprises about 23% of one or more secondary salts selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof by total volume of the hybridization buffer formulation. In some embodiments, the hybridization buffer formulation comprises about 24% of one or more secondary salts selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof by total volume of the hybridization buffer formulation. In some embodiments, the hybridization buffer formulation comprises about 24.5% of one or more secondary salts selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof by total volume of the hybridization buffer formulation. In some embodiments, the hybridization buffer formulation comprises about 25% of one or more secondary salts selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof by total volume of the hybridization buffer formulation. In some embodiments, the hybridization buffer formulation comprises about 26% of one or more secondary salts selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof by total volume of the hybridization buffer formulation. In some embodiments, the hybridization buffer formulation comprises about 28% of one or more secondary salts selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof by total volume of the hybridization buffer formulation. In some embodiments, the hybridization buffer formulation comprises about 28.5% of one or more secondary salts by total volume of the hybridization buffer formulation. In some embodiments, the hybridization buffer formulation comprises about 29% of one or more secondary salts selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof by total volume of the hybridization buffer formulation. In some embodiments, the hybridization buffer formulation comprises about 29.5% of one or more secondary salts selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof by total volume of the hybridization buffer formulation. In some embodiments, the hybridization buffer formulation comprises about 30% of one or more secondary salts by total volume of the hybridization buffer formulation. In some embodiments, the hybridization buffer formulation comprises about 30.5% of one or more secondary salts selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof by total volume of the hybridization buffer formulation. In some embodiments, the hybridization buffer formulation comprises about 31% of one or more secondary salts selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof by total volume of the hybridization buffer formulation.

### Examples of Hybridization Buffers

In some embodiments, the hybridization buffer formulation comprises: (i) between about 31% to about 55% DMSO by total volume of the hybridization buffer formulation; (ii) between about 0.0008% to about 0.0016% of one or more surfactants by total volume of the hybridization buffer formulation; (iii) between about 17% to about 30% of one or more quaternary ammonium salts by total volume of the hybridization buffer formulation; (iv) between about 2% to about 8% of one or more buffers by total volume of the hybridization buffer formulation; and (v) between about 20% to about 35% of one or more secondary salts by total volume of the hybridization buffer formulation. In some embodiments, the one or more surfactants are anionic surfactants. In some embodiments, the surfactant is selected from the group consisting of polysorbate 20, polysorbate 40, polysorbate 60, polysorbate 80, and combinations thereof. some embodiments, the one or more quaternary ammonium salts are selected from the group consisting of tetramethylammonium chloride, tetraethylammonium chloride, tetrabutylammonium chloride, trimethylammonium chloride, Tetra-n-butylammonium chloride, and combinations thereof. In some embodiments, the one or more buffers comprises 2-morpholinoethanesulfonic acid (MES/MES salt). In some embodiments, the one or more secondary salts are selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof.

In other embodiments, the hybridization buffer formulation comprises: (i) between about 36% to about 50% DMSO by total volume of the hybridization buffer formulation; (ii) between about 0.0008% to about 0.0016% of one or more surfactants by total volume of the hybridization buffer formulation; (iii) between about 20% to about 26% of one or more quaternary ammonium salts by total volume of the hybridization buffer formulation; (iv) between about 5% to about 6.5% of one or more buffers by total volume of the hybridization buffer formulation; and (v) between about 25% to about 32% of one or more secondary salts by total volume of the hybridization buffer formulation. In some embodiments, the one or more surfactants are anionic surfactants. In some embodiments, the one or more surfactant is selected from the group consisting of polysorbate 20, polysorbate 40, polysorbate 60, polysorbate 80, and combinations thereof. In some embodiments, the one or more quaternary ammonium salts are selected from the group consisting of tetramethylammonium chloride, tetraethylammonium chloride, tetrabutylammonium chloride, trimethylammonium chloride, Tetra-n-butylammonium chloride, and combinations thereof. In some embodiments, the one or more buffers comprises 2-morpholinoethanesulfonic acid (MES/MES salt). In some embodiments, the one or more secondary salts are selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof.

In others embodiments, the hybridization buffer formulation comprises: (i) between about 48% to about 52% DMSO by total volume of the hybridization buffer formulation; (ii) between about 0.0008% to about 0.0016% of one or more surfactants by total volume of the hybridization buffer formulation; (iii) between about 18% about 22% of one or more quaternary ammonium salts by total volume of the hybridization buffer formulation; (iv) between about 4% about 6% of one or more buffers by total volume of the hybridization buffer formulation; and (v) between 24% about 26% of one or more secondary salts by total volume of the hybridization buffer formulation. In some embodiments, the one or more surfactants are anionic surfactants. In some embodiments, the surfactant is selected from the group consisting of polysorbate 20, polysorbate 40, polysorbate 60, polysorbate 80, and combinations thereof. In some embodiments, the one or more quaternary ammonium salts are selected from the group consisting of tetramethylammonium chloride, tetraethylammonium chloride, tetrabutylammonium chloride, trimethylammonium chloride, Tetra-n-butylammonium chloride, and combinations thereof. In some embodiments, the one or more buffers comprises 2-morpholinoethanesulfonic acid (MES/MES salt). In some embodiments, the one or more secondary salts are selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof.

In others embodiments, the hybridization buffer formulation comprises: (i) between about 38% to about 42% DMSO by total volume of the hybridization buffer formulation; (ii) between about 0.0008% to about 0.0016% of one or more surfactants by total volume of the hybridization buffer formulation; (iii) between about 22% about 26% of one or more quaternary ammonium salts by total volume of the hybridization buffer formulation; (iv) between about 5% about 7% of one or more buffers by total volume of the hybridization buffer formulation; and (v) between 28% about 31% of one or more secondary salts by total volume of the hybridization buffer formulation. In some embodiments, the one or more surfactants are anionic surfactants. In some embodiments, the surfactant is selected from the group consisting of polysorbate 20, polysorbate 40, polysorbate 60, polysorbate 80, and combinations thereof. In some embodiments, the one or more quaternary ammonium salts are selected from the group consisting of tetramethylammonium chloride, tetraethylammonium chloride, tetrabutylammonium chloride, trimethylammonium chloride, Tetra-n-butylammonium chloride, and combinations thereof. In some embodiments, the one or more buffers comprises 2-morpholinoethanesulfonic acid (MES/MES salt). In some embodiments, the one or more secondary salts are selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof.

In others embodiments, the hybridization buffer formulation comprises: (i) between 38% to 42% DMSO by total volume of the hybridization buffer formulation; (ii) between 0.0008% to 0.0016% of one or more surfactants by total volume of the hybridization buffer formulation; (iii) between 22% to 26% of one or more quaternary ammonium salts by total volume of the hybridization buffer formulation; (iv) between 5% to 7% of one or more buffers by total volume of the hybridization buffer formulation; and (v) between 28% to 31% of one or more secondary salts by total volume of the hybridization buffer formulation. In some embodiments, the one or more surfactants are anionic surfactants. In some embodiments, the surfactant is selected from the group consisting of polysorbate 20, polysorbate 40, polysorbate 60, polysorbate 80, and combinations thereof. In some embodiments, the one or more quaternary ammonium salts are selected from the group consisting of tetramethylammonium chloride, tetraethylammonium chloride, tetrabutylammonium chloride, trimethylammonium chloride, Tetra-n-butylammonium chloride, and combinations thereof. In some embodiments, the one or more buffers comprises 2-morpholinoethanesulfonic acid (MES/MES salt). In some embodiments, the one or more secondary salts are selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof.

Yet other examples of suitable hybridization buffer formulations are set forth below in Table 1:

**Table 1: Examples of suitable hybridization buffer formulations.**

| **Component name** | **Formulation Example 1 (% v/v)** | **Formulation Example 2 (% v/v)** |
|---|---|---|
| **Tetramethylammonium chloride** | 20 | 24 |
| **Betaine (aqueous)** | 24.9 | 29.9 |
| **MES/MES salt** | 5 | 6 |
| **Tween-20** | 0.001 | 0.0012 |
| **DMSO** | 50 | 40 |

### REACTION MIXTURES

In another aspect of the present disclosure is a reaction mixture including any of the hybridization buffer formulations (e.g. Formulation Examples 1 and2) described herein and at least one additional component. In some embodiments, the at least one additional component is selected from quaternary ammonium salts, secondary salts, buffers, surfactants, and water.

In some embodiments, the reaction mixture includes any of the disclosed hybridization buffers combined with at least one of: one or more of one or more quaternary ammonium salts, one or more secondary salts, one or more buffers, one or more surfactants, and water. In other embodiments, the reaction mixture includes any of the disclosed hybridization buffers combined with additional amounts of at least two of: one or more of one or more quaternary ammonium salts, one or more secondary salts, one or more buffers, one or more surfactants, and water. In yet other embodiments, the reaction mixture includes any of the disclosed hybridization buffers combined with additional amounts of at least three of: one or more of one or more quaternary ammonium salts, one or more secondary salts, one or more buffers, one or more surfactants, and water. In some embodiments, the reaction mixture is a combination of any of the hybridization buffers described herein and further includes each of: one or more of one or more quaternary ammonium salts, one or more secondary salts, one or more buffers, one or more surfactants, and water.

In some embodiments, the reaction mixtures are able to be stored for between about 3 to about 18 months at a temperature ranging from between about - 20°C to about -15°C without the formation of substantially any precipitates. In other embodiments, reaction mixtures are able to be stored for between about 3 to about 12 months at a temperature ranging from between about -20°C to about -15°C without the formation of substantially any precipitates.

In some embodiments, the reaction mixtures of the present disclosure are capable of being used at temperatures below about 18°C without precipitation of 2-(N-morpholino)ethanesulfonic acid. In some embodiments, the reaction mixtures of the present disclosure are capable of being used at temperatures below about 15°C without precipitation of 2-(N-morpholino)ethanesulfonic acid. In some embodiments, the reaction mixtures of the present disclosure are capable of being used at temperatures below about 10°C without precipitation of 2-(N-morpholino)ethanesulfonic acid. In some embodiments, the reaction mixtures of the present disclosure are capable of being used at temperatures below about 5°C without precipitation of 2-(N-morpholino)ethanesulfonic acid. In some embodiments, the reaction mixtures of the present disclosure are capable of being used at temperatures below about 0°C without precipitation of 2-(N-morpholino)ethanesulfonic acid. In some embodiments, the reaction mixtures of the present disclosure are capable of being used at temperatures below about -5°C without precipitation of 2-(N-morpholino)ethanesulfonic acid. In some embodiments, the reaction mixtures of the present disclosure are capable of being used at temperatures below about -10°C without precipitation of 2-(N-morpholino)ethanesulfonic acid. In some embodiments, the reaction mixtures of the present disclosure are capable of being used at temperatures below about -15°C without precipitation of 2-(N-morpholino)ethanesulfonic acid. In some embodiments, the reaction mixtures of the present disclosure are capable of being used at temperatures below about -20°C without precipitation of 2-(N-morpholino)ethanesulfonic acid. In some embodiments, the reaction mixtures of the present disclosure are capable of being used at temperatures below about -25°C without precipitation of 2-(N-morpholino)ethanesulfonic acid.

In some embodiments, the reaction mixture comprises between about 15% to about 40% DMSO by total volume of the reaction mixture. In some embodiments, the reaction mixture comprises between about 17% to about 39% DMSO by total volume of the reaction mixture. In some embodiments, the reaction mixture comprises between about 19% to about 37% DMSO by total volume of the reaction mixture. In some embodiments, the reaction mixture comprises between about 21% to about 36% DMSO by total volume of the reaction mixture.

In some embodiments, the reaction mixture comprises between about 20% DMSO by total volume of the reaction mixture. In some embodiments, the reaction mixture comprises between about 21% DMSO by total volume of the reaction mixture. In some embodiments, the reaction mixture comprises between about 22% DMSO by total volume of the reaction mixture. In some embodiments, the reaction mixture comprises between about 23% DMSO by total volume of the reaction mixture. In some embodiments, the reaction mixture comprises between about 24% DMSO by total volume of the reaction mixture. In some embodiments, the reaction mixture comprises between about 25% DMSO by total volume of the reaction mixture. In some embodiments, the reaction mixture comprises between about 26% DMSO by total volume of the reaction mixture. In some embodiments, the reaction mixture comprises between about 27% DMSO by total volume of the reaction mixture. In some embodiments, the reaction mixture comprises between about 28% DMSO by total volume of the reaction mixture. In some embodiments, the reaction mixture comprises between about 29% DMSO by total volume of the reaction mixture. In some embodiments, the reaction mixture comprises between about 30% DMSO by total volume of the reaction mixture. In some embodiments, the reaction mixture comprises between about 31% DMSO by total volume of the reaction mixture. In some embodiments, the reaction mixture comprises between about 32% DMSO by total volume of the reaction mixture. In some embodiments, the reaction mixture comprises between about 33% DMSO by total volume of the reaction mixture. In some embodiments, the reaction mixture comprises between about 34% DMSO by total volume of the reaction mixture. In some embodiments, the reaction mixture comprises between about 35% DMSO by total volume of the reaction mixture. In some embodiments, the reaction mixture comprises between about 36% DMSO by total volume of the reaction mixture. In some embodiments, the reaction mixture comprises between about 37% DMSO by total volume of the reaction mixture. In some embodiments, the reaction mixture comprises between about 38% DMSO by total volume of the reaction mixture.

In some embodiments, the reaction mixture comprises between about 0.0008% to about 0.002% of one or more surfactants by total volume of the reaction mixture. In some embodiments, the reaction mixture comprises between about 0.001% to about 0.002% of one or more surfactants by total volume of the reaction mixture. In some embodiments, the reaction mixture comprises between about 0.0011% to about 0.0018% of one or more surfactants by total volume of the reaction mixture. In some embodiments, the reaction mixture comprises about 0.0011% of one or more surfactants by total volume of the reaction mixture. In some embodiments, the reaction mixture comprises about 0.0012% of one or more surfactants by total volume of the reaction mixture. In some embodiments, the reaction mixture comprises between about 0.00125% of one or more surfactants by total volume of the reaction mixture. In some embodiments, the reaction mixture comprises about 0.0013% of one or more surfactants by total volume of the reaction mixture. In some embodiments, the reaction mixture comprises about 0.0014% of one or more surfactants by total volume of the reaction mixture. In some embodiments, the reaction mixture comprises about 0.0015% of one or more surfactants by total volume of the reaction mixture. In some embodiments, the reaction mixture comprises about 0.00155% of one or more surfactants by total volume of the reaction mixture. In some embodiments, the reaction mixture comprises about 0.0016% of one or more surfactants by total volume of the reaction mixture. In some embodiments, the reaction mixture comprises about 0.0017% of one or more surfactants by total volume of the reaction mixture.

In some embodiments, the reaction mixture comprises between 20% about 35% of one or more quaternary ammonium salts by total volume of the reaction mixture. In some embodiments, the reaction mixture comprises between 21% about 34% of one or more quaternary ammonium salts by total volume of the reaction mixture. In some embodiments, the reaction mixture comprises between 22% about 33% of one or more quaternary ammonium salts by total volume of the reaction mixture. In some embodiments, the reaction mixture comprises between 23% about 32% of one or more quaternary ammonium salts by total volume of the reaction mixture. In some embodiments, the reaction mixture comprises between 24% about 32% of one or more quaternary ammonium salts by total volume of the reaction mixture.

In some embodiments, the reaction mixture comprises about 22% of one or more quaternary ammonium salts by total volume of the reaction mixture. In some embodiments, the reaction mixture comprises about 23% of one or more quaternary ammonium salts by total volume of the reaction mixture. In some embodiments, the reaction mixture comprises about 24% of one or more quaternary ammonium salts by total volume of the reaction mixture. In some embodiments, the reaction mixture comprises about 25% of one or more quaternary ammonium salts by total volume of the reaction mixture. In some embodiments, the reaction mixture comprises about 26% of one or more quaternary ammonium salts by total volume of the reaction mixture. In some embodiments, the reaction mixture comprises about 27% of one or more quaternary ammonium salts by total volume of the reaction mixture. In some embodiments, the reaction mixture comprises about 28% of one or more quaternary ammonium salts by total volume of the reaction mixture. In some embodiments, the reaction mixture comprises about 29% of one or more quaternary ammonium salts by total volume of the reaction mixture. In some embodiments, the reaction mixture comprises about 30% of one or more quaternary ammonium salts by total volume of the reaction mixture. In some embodiments, the reaction mixture comprises about 31% of one or more quaternary ammonium salts by total volume of the reaction mixture. In some embodiments, the reaction mixture comprises about 32% of one or more quaternary ammonium salts by total volume of the reaction mixture. In some embodiments, the reaction mixture comprises about 33% of one or more quaternary ammonium salts by total volume of the reaction mixture.

In some embodiments, the reaction mixture comprises between 3% about 10% of one or more buffers by total volume of the reaction mixture. In some embodiments, the reaction mixture comprises between 4% about 10% of one or more buffers by total volume of the reaction mixture. In some embodiments, the reaction mixture comprises between 4% about 9% of one or more buffers by total volume of the reaction mixture. In some embodiments, the reaction mixture comprises between 5% about 9% of one or more buffers by total volume of the reaction mixture. In some embodiments, the reaction mixture comprises between 5% about 8% of one or more buffers by total volume of the reaction mixture. In some embodiments, the reaction mixture comprises between 6% about 8% of one or more buffers by total volume of the reaction mixture.

In some embodiments, the reaction mixture comprises about 5% of one or more buffers by total volume of the reaction mixture. In some embodiments, the reaction mixture comprises about 5.5% of one or more buffers by total volume of the reaction mixture. In some embodiments, the reaction mixture comprises about 6% of one or more buffers by total volume of the reaction mixture. In some embodiments, the reaction mixture comprises about 6.5% of one or more buffers by total volume of the reaction mixture. In some embodiments, the reaction mixture comprises about 7% of one or more buffers by total volume of the reaction mixture. In some embodiments, the reaction mixture comprises about 7.5% of one or more buffers by total volume of the reaction mixture. In some embodiments, the reaction mixture comprises about 8% of one or more buffers by total volume of the reaction mixture. In some embodiments, the reaction mixture comprises about 8.5% of one or more buffers by total volume of the reaction mixture.

In some embodiments, the reaction mixture comprises between 26% about 44% of one or more secondary salts by total volume of the reaction mixture. In some embodiments, the reaction mixture comprises between 27% about 43% of one or more secondary salts by total volume of the reaction mixture. In some embodiments, the reaction mixture comprises between 28% about 42% of one or more secondary salts by total volume of the reaction mixture. In some embodiments, the reaction mixture comprises between 29% about 41% of one or more secondary salts by total volume of the reaction mixture. In some embodiments, the reaction mixture comprises between 30% about 40% of one or more secondary salts by total volume of the reaction mixture.

In some embodiments, the reaction mixture comprises about 28% of one or more secondary salts by total volume of the reaction mixture. In some embodiments, the reaction mixture comprises about 30% of one or more secondary salts by total volume of the reaction mixture. In some embodiments, the reaction mixture comprises about 32% of one or more secondary salts by total volume of the reaction mixture. In some embodiments, the reaction mixture comprises about 34% of one or more secondary salts by total volume of the reaction mixture. In some embodiments, the reaction mixture comprises about 36% of one or more secondary salts by total volume of the reaction mixture. In some embodiments, the reaction mixture comprises about 38% of one or more secondary salts by total volume of the reaction mixture. In some embodiments, the reaction mixture comprises about 40% of one or more secondary salts by total volume of the reaction mixture. In some embodiments, the reaction mixture comprises about 42% of one or more secondary salts by total volume of the reaction mixture.

In some embodiments, the reaction mixture comprises between 0% about 0.05% of water by total volume of the reaction mixture. In some embodiments, the reaction mixture comprises between 0% about 0.04% of water by total volume of the reaction mixture. In some embodiments, the reaction mixture comprises between 0% about 0.03% of water by total volume of the reaction mixture. In some embodiments, the reaction mixture comprises between 0% about 0.02% of water by total volume of the reaction mixture. In some embodiments, the reaction mixture comprises between 0% about 0.01% of water by total volume of the reaction mixture.

### Examples of Reaction Mixtures

In other embodiments, the reaction mixture comprises: (i) between about 18% to about 38% DMSO by total volume of the reaction mixture; (ii) between about 0.0008% to about 0.002% of one or more surfactants by total volume of the reaction mixture; (iii) between about 22% to about 33% of one or more quaternary ammonium salts by total volume of the reaction mixture; (iv) between about 5% to about 8% of one or more buffers by total volume of the reaction mixture; (v) between about 28% to about 42% of one or more secondary salts by total volume of the reaction mixture; and (vi) between about 0% to about 0.04% water. In some embodiments, the one or more surfactants are anionic surfactants. In some embodiments, the surfactant is selected from the group consisting of polysorbate 20, polysorbate 40, polysorbate 60, polysorbate 80, and combinations thereof. In some embodiments, the one or more quaternary ammonium salts are selected from the group consisting of tetramethylammonium chloride, tetraethylammonium chloride, tetrabutylammonium chloride, trimethylammonium chloride, Tetra-n-butylammonium chloride, and combinations thereof. In some embodiments, the one or more buffers comprises 2-morpholinoethanesulfonic acid (MES/MES salt). In some embodiments, the one or more secondary salts are selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof.

In yet other embodiments, the reaction mixture comprises: (i) between about 21% to about 36% DMSO by total volume of the reaction mixture; (ii) between about 0.0008% to about 0.002% of one or more surfactants by total volume of the reaction mixture; (iii) between about 24% to about 32% of one or more quaternary ammonium salts by total volume of the reaction mixture; (iv) between about 5% to about 8% of one or more buffers by total volume of the reaction mixture; (v) between about 28% to about 42% of one or more secondary salts by total volume of the reaction mixture; and (vi) between about 0% to about 0.04% water. In some embodiments, the one or more surfactants are anionic surfactants. In some embodiments, the surfactant is selected from the group consisting of polysorbate 20, polysorbate 40, polysorbate 60, polysorbate 80, and combinations thereof. In some embodiments, the one or more quaternary ammonium salts are selected from the group consisting of tetramethylammonium chloride, tetraethylammonium chloride, tetrabutylammonium chloride, trimethylammonium chloride, Tetra-n-butylammonium chloride, and combinations thereof. In some embodiments, the one or more buffers comprises 2-morpholinoethanesulfonic acid (MES/MES salt). In some embodiments, the one or more secondary salts are selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof.

In yet other embodiments, the reaction mixture comprises: (i) between 21% to 36% DMSO by total volume of the reaction mixture; (ii) between 0.0008% to 0.002% of one or more surfactants by total volume of the reaction mixture; (iii) between 24% to 32% of one or more quaternary ammonium salts by total volume of the reaction mixture; (iv) between 5% to 8% of one or more buffers by total volume of the reaction mixture; (v) between 28% to 42% of one or more secondary salts by total volume of the reaction mixture; and (vi) between 0% to 0.04% water. In some embodiments, the one or more surfactants are anionic surfactants. In some embodiments, the surfactant is selected from the group consisting of polysorbate 20, polysorbate 40, polysorbate 60, polysorbate 80, and combinations thereof. In some embodiments, the one or more quaternary ammonium salts are selected from the group consisting of tetramethylammonium chloride, tetraethylammonium chloride, tetrabutylammonium chloride, trimethylammonium chloride, Tetra-n-butylammonium chloride, and combinations thereof. In some embodiments, the one or more buffers comprises 2-morpholinoethanesulfonic acid (MES/MES salt). In some embodiments, the one or more secondary salts are selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof.

Yet other examples of suitable reaction mixtures are set forth below:

**Table 2: Examples of reaction mixtures**

| **Component name** | **Formulation Example 3 (% v/v)** | **Formulation Example 4 (% v/v)** |
|---|---|---|
| **Tetramethylammonium chloride** | 24 | 24 |
| **Betaine (6M aqueous)** | 30 | 38 |
| **MES/MES salt** | 6 | 7.6 |
| **Tween-20** | 0.0012 | 0.00152 |
| **water** | 0.04 | 0.02 |
| **DMSO** | 35.95 | 21.57 |

### MASTER MIXES

In another aspect of the present disclosure is a master mix comprising (i) any one of the reaction mixtures described herein (e.g. Formulation Examples 3 and 4); and (ii) one or more oligonucleotides.

In some embodiments, the master mixture comprises between about 60% and about 80% of any one of the reaction mixtures described herein. In some embodiments, the master mixture comprises between about 62% and about 78% of any one of the reaction mixtures described herein, with the remainder of the master mix comprising oligonucleotides or mixtures of oligonucleotides. In some embodiments, the master mixture comprises between about 64% and about 80% of any one of the reaction mixtures described herein, with the remainder of the master mix comprising oligonucleotides or mixtures of oligonucleotides. In some embodiments, the master mixture comprises between about 76% and about 80% of any one of the reaction mixtures described herein, with the remainder of the master mix comprising oligonucleotides or mixtures of oligonucleotides. In some embodiments, the master mixture comprises between about 66% and about 80% of any one of the reaction mixtures described herein, with the remainder of the master mix comprising oligonucleotides or mixtures of oligonucleotides. In some embodiments, the master mixture comprises between about 74% and about 80% of any one of the reaction mixtures described herein, with the remainder of the master mix comprising oligonucleotides or mixtures of oligonucleotides. In some embodiments, the master mixture comprises between about 68% and about 72% of any one of the reaction mixtures described herein, with the remainder of the master mix comprising oligonucleotides or mixtures of oligonucleotides. In some embodiments, the master mixture comprises about 60% of any one of the reaction mixtures described herein, with the remainder of the master mix comprising oligonucleotides or mixtures of oligonucleotides.

In some embodiments, the master mixture comprises between 60% and 80% of any one of the reaction mixtures described herein. In some embodiments, the master mixture comprises between 62% and 78% of any one of the reaction mixtures described herein, with the remainder of the master mix comprising oligonucleotides or mixtures of oligonucleotides. In some embodiments, the master mixture comprises between 64% and 80% of any one of the reaction mixtures described herein, with the remainder of the master mix comprising oligonucleotides or mixtures of oligonucleotides. In some embodiments, the master mixture comprises between 76% and 80% of any one of the reaction mixtures described herein, with the remainder of the master mix comprising oligonucleotides or mixtures of oligonucleotides. In some embodiments, the master mixture comprises between 66% and 80% of any one of the reaction mixtures described herein, with the remainder of the master mix comprising oligonucleotides or mixtures of oligonucleotides. In some embodiments, the master mixture comprises between 74% and 80% of any one of the reaction mixtures described herein, with the remainder of the master mix comprising oligonucleotides or mixtures of oligonucleotides. In some embodiments, the master mixture comprises between 68% and 72% of any one of the reaction mixtures described herein, with the remainder of the master mix comprising oligonucleotides or mixtures of oligonucleotides. In some embodiments, the master mixture comprises 60% of any one of the reaction mixtures described herein, with the remainder of the master mix comprising oligonucleotides or mixtures of oligonucleotides.

In some embodiments, the master mixture consists essentially of between about 60% and about 80% of any one of the reaction mixtures described herein. In some embodiments, the master mixture consists essentially of between about 62% and about 78% of any one of the reaction mixtures described herein, with the remainder of the master mix comprising oligonucleotides or mixtures of oligonucleotides. In some embodiments, the master mixture consists essentially of between about 64% and about 80% of any one of the reaction mixtures described herein, with the remainder of the master mix comprising oligonucleotides or mixtures of oligonucleotides. In some embodiments, the master mixture consists essentially of between about 76% and about 80% of any one of the reaction mixtures described herein, with the remainder of the master mix comprising oligonucleotides or mixtures of oligonucleotides. In some embodiments, the master mixture consists essentially of between about 66% and about 80% of any one of the reaction mixtures described herein, with the remainder of the master mix comprising oligonucleotides or mixtures of oligonucleotides. In some embodiments, the master mixture consists essentially of between about 74% and about 80% of any one of the reaction mixtures described herein, with the remainder of the master mix comprising oligonucleotides or mixtures of oligonucleotides. In some embodiments, the master mixture consists essentially of between about 68% and about 72% of any one of the reaction mixtures described herein, with the remainder of the master mix comprising oligonucleotides or mixtures of oligonucleotides. In some embodiments, the master mixture consists essentially of about 60% of any one of the reaction mixtures described herein, with the remainder of the master mix comprising oligonucleotides or mixtures of oligonucleotides.

In some embodiments, the master mixture consists of between about 60% and about 80% of any one of the reaction mixtures described herein. In some embodiments, the master mixture consists of between about 62% and about 78% of any one of the reaction mixtures described herein, with the remainder of the master mix comprising oligonucleotides or mixtures of oligonucleotides. In some embodiments, the master mixture consists of between about 64% and about 80% of any one of the reaction mixtures described herein, with the remainder of the master mix comprising oligonucleotides or mixtures of oligonucleotides. In some embodiments, the master mixture consists of between about 76% and about 80% of any one of the reaction mixtures described herein, with the remainder of the master mix comprising oligonucleotides or mixtures of oligonucleotides. In some embodiments, the master mixture consists of between about 66% and about 80% of any one of the reaction mixtures described herein, with the remainder of the master mix comprising oligonucleotides or mixtures of oligonucleotides. In some embodiments, the master mixture consists of between about 74% and about 80% of any one of the reaction mixtures described herein, with the remainder of the master mix comprising oligonucleotides or mixtures of oligonucleotides. In some embodiments, the master mixture consists of between about 68% and about 72% of any one of the reaction mixtures described herein, with the remainder of the master mix comprising oligonucleotides or mixtures of oligonucleotides. In some embodiments, the master mixture consists of about 60% of any one of the reaction mixtures described herein, with the remainder of the master mix comprising oligonucleotides or mixtures of oligonucleotides.

In some embodiments, the master mixture consists of between 60% and 80% of any one of the reaction mixtures described herein. In some embodiments, the master mixture consists of between 62% and 78% of any one of the reaction mixtures described herein, with the remainder of the master mix comprising oligonucleotides or mixtures of oligonucleotides. In some embodiments, the master mixture consists of between 64% and 80% of any one of the reaction mixtures described herein, with the remainder of the master mix comprising oligonucleotides or mixtures of oligonucleotides. In some embodiments, the master mixture consists of between 76% and 80% of any one of the reaction mixtures described herein, with the remainder of the master mix comprising oligonucleotides or mixtures of oligonucleotides. In some embodiments, the master mixture consists of between 66% and 80% of any one of the reaction mixtures described herein, with the remainder of the master mix comprising oligonucleotides or mixtures of oligonucleotides. In some embodiments, the master mixture consists of between 74% and 80% of any one of the reaction mixtures described herein, with the remainder of the master mix comprising oligonucleotides or mixtures of oligonucleotides. In some embodiments, the master mixture consists of between 68% and 72% of any one of the reaction mixtures described herein, with the remainder of the master mix comprising oligonucleotides or mixtures of oligonucleotides. In some embodiments, the master mixture consists of 60% of any one of the reaction mixtures described herein, with the remainder of the master mix comprising oligonucleotides or mixtures of oligonucleotides.

In some embodiments, the oligonucleotides include probes, nucleic acids from an obtained sample (or fragments of nucleic acids from an obtained sample) and blocking oligonucleotides. In some embodiments, the oligonucleotides include one or more oligonucleotide probes, such as a pool of oligonucleotide probes. In some embodiments, the oligonucleotide probes are reference populations of nucleic acid sequences capable of hybridizing to complementary nucleic acid sequences within a genomic sample or a population of nucleic acid fragments (such as nucleic acid fragments generated from an obtained genomic sample). In some embodiments, the oligonucleotide probes are designed to target desired genes, exons, and/or other genomic regions of interest within a genomic sample or a population of nucleic acid fragments. In some embodiments, the oligonucleotide probes are selected such that the oligonucleotide probes relate to, by way of non-limiting examples, a set of genes of interest, all of the exons of a genome, particular genetic regions of interest, disease or physiological states and the like.

In some embodiments, the oligonucleotide probes are DNA capture probes. In some embodiments, the DNA capture probes include a pool of Roche SeqCap EZ Probes (available from Roche Sequencing and Life Sciences, Indianapolis, IND). In some embodiments, a pool of Roche SeqCap EZ Probes include a mixture of different biotinylated single-stranded DNA oligonucleotides in solution, each with a specific sequence, where the length of individual oligonucleotides can range from about 50 nucleotides to about 100 nucleotides with a typical size of about 75 nucleotides. In some embodiments, a Roche SeqCap EZ Probe Pool can be used in sequence capture experiments to hybridize to targeted complementary fragments of a DNA sequencing library and thus to capture and enrich them relative to untargeted fragments of the same DNA sequencing library prior to sequencing. The DNA sequencing library may be constructed from genomic DNA for genome analysis, or from cDNA prepared from RNA or mRNA for transcriptome analysis, and it may be constructed from the DNA or cDNA of any species of organism from which these nucleic acids can be extracted.

As used herein, a "blocking oligonucleotide" is an engineered, single stranded nucleic acid sequence. In some embodiments, the blocking oligonucleotide may be one of single stranded DNA, RNA, peptide nucleic acid or locked nucleic acid. Preferably it is a DNA oligonucleotide. In some embodiments, the blocking oligonucleotide generally comprises from 10 to 40 nucleotides. In other embodiments, the blocking oligonucleotide comprises between about 15 to 30 nucleotides. Examples of suitable blocking oligonucleotides are described in U.S. Publication Nos. 2016/0076089 and 2016/0177; 72.

In some embodiments, the oligonucleotides include nucleic acids from an obtained sample, such as an obtained genomic sample. In some embodiments, the obtained genomic sample is a sample derived from a mammalian subject, e.g. a human subject. In some embodiments, the obtained genomic sample is a blood sample, or a blood plasma sample obtained from a mammalian subject, e.g. a blood sample or a blood plasma sample obtained from a human subject. In some embodiments, the obtained genomic sample is in the form of cell-free nucleic acids. In some embodiments, the obtained genomic sample in the form of cell-free nucleic acids comprises DNA and/or RNA. In some embodiments, the cell-free DNA typically ranges in size from between about 200 bp to about 130 bp. In some embodiments, the cell-free DNA typically ranges in size from between about 190 bp to about 140 bp. In some embodiments, the cell-free DNA typically ranges in size from between about 180 bp to about 150 bp. Non-limiting examples of cell-free nucleic acids include circulating tumor DNA (ctDNA) and fetal cell-free DNA present in maternal blood and blood plasma. In some embodiments, the present disclosure also encompasses isolation of various types of cell-free RNA.

In some embodiments, the obtained genomic sample comprises target and non-target nucleic acid sequences. As used herein, the term "target nucleic acid" refers to a nucleic acid whose presence is to be detected, measured, amplified, and/or subject to further assays and analyses. A target nucleic acid may comprise any single and/or double-stranded nucleic acid. Target nucleic acids can exist as isolated nucleic acid fragments or be a part of a larger nucleic acid fragment. Target nucleic acids can be derived or isolated from essentially any source, such as cultured microorganisms, uncultured microorganisms, complex biological mixtures, biological samples, tissues, sera, ancient or preserved tissues or samples, environmental isolates or the like. Further, target nucleic acids include or are derived from cDNA, RNA, genomic DNA, cloned genomic DNA, genomic DNA libraries, enzymatically fragmented DNA or RNA, chemically fragmented DNA or RNA, physically fragmented DNA or RNA, or the like. In some embodiments, a target nucleic acid may comprise a whole genome. In exemplary embodiments, a target nucleic acid may comprise the entire nucleic acid content of a sample and/or biological sample. In exemplary embodiments, a target nucleic acid may comprise circulating or cell-free DNA's, e.g., circulating tumor DNA ("ctDNA") present in individuals with cancer or circulating fetal or circulating maternal DNA ("cfDNA") fragments present in plasma or serum of pregnant women. Target nucleic acids can come in a variety of different forms including, for example, simple or complex mixtures, or in substantially purified forms. For example, a target nucleic acid can be part of a sample that contains other components or can be the sole or major component of the sample. Also, a target nucleic acid can have either a known or unknown sequence.

In some embodiments, the obtained genomic sample comprises fragmented nucleic acid sequences. In some embodiments, the generated nucleic acid fragments have a length which are less than about 1000 base pairs. In other embodiments, the generated nucleic acid fragments comprise sequence fragments having a sequence size ranging from between about 100 to about 1000 base pairs in length. In yet other embodiments, the generated nucleic acid fragments comprise sequence fragments having a sequence size ranging from between about 500 to about 750 base pairs in length.

### KITS

Another aspect of the present disclosure is a kit comprising: (i) any of the hybridization buffer formulations described herein (e.g. Formulation Examples 1 and 2), and (ii) a plurality of beads. In some embodiments, the beads are magnetic beads. In other embodiments, the beads are non-magnetic beads. Examples of suitable non-magnetic beads include silica beads, alginate hydrogel beads, agarose hydrogel beads, poly(N-isopropylacrylamide) (NIPAM) gel beads, cellulose beads, polyethylene (PE) beads, polypropylene (PP) beads, polymethyl methacrylate (PMMA) beads, nylon (PA) beads, polyurethane beads, acrylates copolymer beads, polyquaterniums beads, polysorbate beads, and polyethylene glycol (PEG) beads.

Another aspect of the present disclosure is a kit comprising: (i) any of the reaction mixtures described herein (e.g. Formulation Examples 3 and 4), and (ii) a plurality of beads. In some embodiments, the beads are magnetic beads. In other embodiments, the beads are non-magnetic beads

Yet another aspect of the present disclosure is a kit comprising: (i) any of the master mixes described herein, and (ii) a plurality of beads. In some embodiments, the beads are magnetic beads. In other embodiments, the beads are non-magnetic beads

An even further aspect of the present disclosure comprises (i) any of the reaction mixtures described herein; and (ii) a dPCR chip. In some embodiments, dPCR chip may include, for example, a silicon substrate etched with nano-scale or smaller reaction wells. In some embodiments, a dPCR chip has a low thermal mass. For example, the chip may be constructed of thin, highly conductive materials that do not store heat energy. In some embodiments, a dPCR chip has a surface area of from about 50 mm² to about 150 mm². In some embodiments a dPCR chip has a surface area of about 100 mm². Limiting the surface area may allow for greater uniformity of heating of the chip during melt analysis and a reduction in run-to-run variation in the melt cure analysis, a reduction in errors in melt curve generation, and increased discrimination of melt curves in the analysis. Other dPCR chips are describes in PCT Publication No. WO/2016/133783,

### TARGET ENRICHMENT METHODS

The present disclosure also relates to a method of reducing the complexity of a nucleic acid sample by enriching for one or more specific nucleic acid target sequences within the nucleic acid sample, wherein the method utilizes any of the formulations described herein, such as any of the hybridization buffer or reaction mixture formulations described herein. In some embodiments, the present disclosure is directed to methods of enriching for one or more specific target sequences in a nucleic acid sample using libraries of oligonucleotide probes. In some embodiments, the nucleic acid sample enriched for the specific target sequences may then be used in downstream sequencing operations. In some embodiments, the method of enriching for specific nucleic acid target sequences includes one or more heat denaturing and hybridization steps. In some embodiments, the one or more heat denaturing and/or hybridization steps of target enrichment may utilize any of the hybridization buffers, reaction mixtures, or master mixes described herein. Examples of systems and method of target enrichment are disclosed in US Publication Nos. 2018/0016630, 2020/0048694, 2017/0037459, and 2018/00871 08.

By way of example, and in some embodiments, target enrichment includes obtaining a genomic sample. In some embodiments, the obtained genomic sample is a sample derived from a mammalian subject, e.g. a human subject. In some embodiments, the obtained genomic sample is a blood sample, or a blood plasma sample obtained from a mammalian subject, e.g. a blood sample or a blood plasma sample obtained from a human subject. In some embodiments, the obtained genomic sample is in the form of cell-free nucleic acids. In some embodiments, the obtained genomic sample in the form of cell-free nucleic acids comprises DNA and/or RNA. In some embodiments, the cell-free DNA typically ranges in size from between about 200 bp to about 130 bp. In some embodiments, the cell-free DNA typically ranges in size from between about 190 bp to about 140 bp. In some embodiments, the cell-free DNA typically ranges in size from between about 180 bp to about 150 bp. Non-limiting examples of cell-free nucleic acids include circulating tumor DNA (ctDNA) and fetal cell-free DNA present in maternal blood and blood plasma. In some embodiments, the present disclosure also encompasses isolation of various types of cell-free RNA.

Alternatively, in some embodiments, target enrichment includes obtaining a genomic sample, e.g. a genomic DNA sample acquired from a human patient. In some embodiments, the obtained genomic sample is sheared into fragments to provide a population of nucleic acid fragments. In some embodiments, shearing of the obtained genomic sample is effectuated using mechanical (e.g. nebulization or sonication) and/or enzymatic fragmentation (e.g. restriction endonucleases).

In some embodiments, the generated nucleic acid fragments are randomly sized. In some embodiments, the generated nucleic acid fragments have a length which are less than about 1000 base pairs. In other embodiments, the generated nucleic acid fragments comprise sequence fragments having a sequence size ranging from between about 100 to about 1000 base pairs in length. In yet other embodiments, the generated nucleic acid fragments comprise sequence fragments having a sequence size ranging from between about 500 to about 750 base pairs in length. In some embodiments, adapters, such as those including a specific barcode sequence, are then added via a ligation reaction to the population of nucleic acid.

Following the obtaining of the genomic sample (and/or the optional fragmentation of the obtained genomic sample), in some embodiments a pool of oligonucleotide probes, such as oligonucleotide probes conjugated to a first member of a pair of specific binding entities, are introduced to the obtained genomic sample or the population of nucleic acid fragments. In some embodiments, the pool of oligonucleotide probes is introduced to a buffer solution, a hybridization buffer solution (including any of those described herein), or a reaction mixture (including any of those described herein) including the obtained genomic sample or the population of nucleic acid fragments. In some embodiments, the oligonucleotide probes are reference populations of nucleic acid sequences capable of hybridizing to complementary nucleic acid sequences within the genomic sample or the population nucleic acid fragments. In some embodiments, the oligonucleotide probes are designed to target desired genes, exons, and/or other genomic regions of interest within the genomic sample or the population of nucleic acid fragments. In some embodiments, the oligonucleotide probes are selected such that the oligonucleotide probes relate to, by way of non-limiting examples, a set of genes of interest, all of the exons of a genome, particular genetic regions of interest, disease or physiological states and the like.

In some embodiments, the oligonucleotide probes are DNA capture probes. In some embodiments, the DNA capture probes include a pool of Roche SeqCap EZ Probes (available from Roche Sequencing and Life Sciences, Indianapolis, IND). In some embodiments, a pool of Roche SeqCap EZ Probes include a mixture of different biotinylated single-stranded DNA oligonucleotides in solution, each with a specific sequence, where the length of individual oligonucleotides can range from about 50 nucleotides to about 100 nucleotides with a typical size of about 75 nucleotides. In some embodiments, a Roche SeqCap EZ Probe Pool can be used in sequence capture experiments to hybridize to targeted complementary fragments of a DNA sequencing library and thus to capture and enrich them relative to untargeted fragments of the same DNA sequencing library prior to sequencing. The DNA sequencing library may be constructed from genomic DNA for genome analysis, or from cDNA prepared from RNA or mRNA for transcriptome analysis, and it may be constructed from the DNA or cDNA of any species of organism from which these nucleic acids can be extracted.

In some embodiments, the oligonucleotide probes hybridize to a first subset of complementary nucleic acids within the genomic sample or nucleic acid fragments within the population of nucleic acid fragments which include the desired genes, exons, and/or other genomic regions of interest to form target-probe complexes having a first member of a pair of specific binding entities. In some embodiments, a second subset of nucleic acids or nucleic acid fragments within the obtained genomic sample or the solution of nucleic acid fragments, respectively, that do not include the desired genes, exons, and/or other genomic regions of interest do not form target-probe complexes and are referred to as "off-target nucleic acids" or "off-target fragments." As such, following the introduction of the oligonucleotide probes, any solution for enrichment may include formed target-probe complexes, off-target nucleic acids or off-target fragments, and/or free probes (assuming that an excess amount of oligonucleotide probes are provided to any solution including adapter-ligated DNA fragments). In some embodiments, the solution for enrichment is provided in a buffer solution.

Subsequently, the solution for enrichment, including the formed target-probe complexes, off-target nucleic acids and/or off-target fragments, are introduced to a device (or a chamber within a device) that is pre-loaded with a plurality of beads, such as magnetic beads, e.g. between about 10 and about 10,000 beads). In some embodiments, the beads are functionalized such that a first reactive group on the oligonucleotide probe binds with a second reactive group of the bead. In this manner, the target-probe complexes become bound to the beads, and thus become immobilized within the device (or a chamber within a device).

Following the binding of the target-probe complexes to the functionalized beads and/or the binding of free-probes to the beads, unbound off-target nucleic acids, off-target fragments, reagents, and/or impurities are then removed from the device (or the chamber of the device). In some embodiments, the unbound nucleic acids are removed by washing, e.g. washing with one or more buffer solutions. In some embodiments, removal of the off-target fragments that were not complementary to any the oligonucleotide probes introduced to the solution for enrichment enriches the remaining immobilized target genomic material.

Following the removal of substantially all off-target nucleic acids, off-target fragments, reagents, and/or impurities from the device (or a chamber of the device), target molecules are removed (i.e. released from the beads) and subsequently collected. In some embodiments, the target molecules or target molecule complexes are released by flowing a fluid or reagent into the device (or the chamber of the device) suitable for releasing the target molecule or the target molecule complex from the particle or bead. In some embodiments, the target molecules are removed from the chamber by flowing a heated fluid through the processing conduit.

For example, a pre-heated fluid may be introduced to the device (or the chamber of the device) to effectuate release. In some embodiments, the temperature-of the pre-heated fluid may range from between about 4°C to about 150°C. In other embodiments, the temperature-of the pre-heated fluid may range from between about 20°C to about 95°C. In yet other embodiments, the temperature-of the pre-heated fluid may range from between about 37°C to about 65°C. In some embodiments, the heated fluid permits the denaturation of the target-probe complexes. In some embodiments, the fluid is a heated buffer. Non-liming examples of buffers include citric acid, potassium dihydrogen phosphate, boric acid, diethyl barbituric acid, piperazine-N,N'-bis(2-ethanesulfonic acid), dimethylarsinic acid, 2-(N-morpholino)ethanesulfonic acid, tris(hydroxymethyl)methylamine (TRIS), 2-(N-morpholino)ethanesulfonic acid (TAPS), N,N-bis(2-hydroxyethyl)glycine(Bicine), N-tris(hydroxymethyl)methylglycine (Tricine), 4-2-hydroxyethyl-1-piperazineethanesulfonic acid (HEPES), 2-{[tris(hydroxymethyl)methyl]amino}ethanesulfonic acid (TES), and combinations thereof. In some embodiments, the unmasking agent is water. In other embodiments, the buffer solution may be comprised of tris(hydroxymethyl)methylamine (TRIS), 2-(N-morpholino)ethanesulfonic acid (TAPS), N,N-bis(2-hydroxyethyl)glycine(Bicine), N -tris(hydroxymethyl)methylglycine (Tricine), 4-2-hydroxyethyl-1-piperazineethanesulfonic acid (HEPES), 2-{[tris(hydroxymethyl)methyl]amino}ethanesulfonic acid (TES), or a combination thereof. In some embodiments, the buffer has a pH ranging from about 5 to about 9.

In other embodiments, a reagent (e.g. an enzyme) is introduced to effectuate release. Examples of suitable enzymes include trypsin (which cleaves the peptide bonds at the carboxyl end of lysine and arginine residues) and clostripain (which cleaves at the carboxyl side of arginine residues).

The released target molecules may then be used in one or more downstream processes, e.g. sequencing, amplification, one or more further chemical reactions, etc. In some embodiments, sequencing may be performed according to any method known to those of ordinary skill in the art. In some embodiments, sequencing methods include Sanger sequencing and dye-terminator sequencing, as well as next-generation sequencing technologies. Instruments and methods of sequencing are disclosed, for example, in PCT Publication Nos. WO2014144478, WO2015058093, WO2014106076 andWO2013068 528.

The term "next generation sequencing" refers to sequencing technologies having high-throughput sequencing as compared to traditional Sanger- and capillary electrophoresis-based approaches, wherein the sequencing process is performed in parallel, for example producing thousands or millions of relatively small sequence reads at a time. Some examples of next generation sequencing techniques include, but are not limited to, sequencing by synthesis, sequencing by ligation, and sequencing by hybridization. These technologies produce shorter reads (anywhere from about 25 - about 500 bp) but many hundreds of thousands or millions of reads in a relatively short time.

Examples of such sequencing devices available from Illumina (San Diego, CA) include, but are not limited to iSEQ, MiniSEQ, MiSEQ, NextSEQ, NoveSEQ. It is believed that the Illumina next-generation sequencing technology uses clonal amplification and sequencing by synthesis (SBS) chemistry to enable rapid sequencing. The process simultaneously identifies DNA bases while incorporating them into a nucleic acid chain. Each base emits a unique fluorescent signal as it is added to the growing strand, which is used to determine the order of the DNA sequence.

A non-limiting example of a sequencing device available from ThermoFisher Scientific (Waltham, MA) includes the Ion Personal Genome Machine^{™} (PGM^{™}) System. It is believed that Ion Torrent sequencing measures the direct release of H+ (protons) from the incorporation of individual bases by DNA polymerase. A non-limiting example of a sequencing device available from Pacific Biosciences (Menlo Park, CA) includes the PacBio Sequel Systems. A non-limiting example of a sequencing device available from Roche (Pleasanton, CA) is the Roche 454.

Next-generation sequencing methods may also include nanopore sequencing methods. In general, three nanopore sequencing approaches have been pursued: strand sequencing in which the bases of DNA are identified as they pass sequentially through a nanopore, exonuclease-based nanopore sequencing in which nucleotides are enzymatically cleaved one-by-one from a DNA molecule and monitored as they are captured by and pass through the nanopore, and a nanopore sequencing by synthesis (SBS) approach in which identifiable polymer tags are attached to nucleotides and registered in nanopores during enzyme-catalyzed DNA synthesis. Common to all these methods is the need for precise control of the reaction rates so that each base is determined in order.

Strand sequencing requires a method for slowing down the passage of the DNA through the nanopore and decoding a plurality of bases within the channel; ratcheting approaches, taking advantage of molecular motors, have been developed for this purpose. Exonuclease-based sequencing requires the release of each nucleotide close enough to the pore to guarantee its capture and its transit through the pore at a rate slow enough to obtain a valid ionic current signal. In addition, both of these methods rely on distinctions among the four natural bases, two relatively similar purines and two similar pyrimidines. The nanopore SBS approach utilizes synthetic polymer tags attached to the nucleotides that are designed specifically to produce unique and readily distinguishable ionic current blockade signatures for sequence determination. In some embodiments, sequencing of nucleic acids comprises via nanopore sequencing comprises: preparing nanopore sequencing complexes and determining polynucleotide sequences. Methods of preparing nanopores and nanopore sequencing are described in U.S. Patent Application Publication No. 2017/0268052, and PCT Publication Nos. WO2014/074727, WO2006/028508, WO2012/083249, and WO/20 14/0747727. In some embodiments, tagged nucleotides may be used in the determination of the polynucleotide sequences (see, e.g., PCT Publication No. WO/2020/131759, WO/2013/191793, and WO/2015/1248402.

In some embodiments, any one of the kits of the present disclosure may include software for analyzing obtained sequencing data. Analysis of the data generated by sequencing is generally performed using software and/or statistical algorithms that perform various data conversions, e.g., conversion of signal emissions into base calls, conversion of base calls into consensus sequences for a nucleic acid template, etc. Such software, statistical algorithms, and the use of such are described in detail, in U.S. Patent Application Publication Nos. 2009/0024331 2017/0044606 and in PCT Publication No. WO/2018/034745

One method of amplifying a target sequence is with a polymerase mediated technique called polymerase chain reaction (PCR). In general, PCR is a method for increasing the concentration of a segment of a target sequence in a mixture of genomic DNA without cloning or purification. Polymerase chain reaction ("PCR") is described, for example, in U.S. Pat. No. 4,683,202; U.S. Pat. No. 4,683,195; U.S. Pat. No. 4,000,159; U.S. Pat. No. 4,965,188; U.S. Pat. No. 5,1 76995. Examples of PCR techniques that can be used include, but are not limited to, quantitative PCR, quantitative fluorescent PCR (QF-PCR), multiplex fluorescent PCR (MF-PCR), real time PCR (RT-PCR), single cell PCR, restriction fragment length polymorphism PCR (PCR-RFLP), PCR-RFLP/RT-PCR-RFLP, hot start PCR, nested PCR, in situ polony PCR, in situ rolling circle amplification (RCA), bridge PCR, picotiter PCR, digital PCR, droplet digital PCR, and emulsion PCR. Droplet and digital droplet PCR systems are further described in U.S. Patent No, 9,822,393 and 10,676,778, the disclosures of which are hereby incorporated by reference herein in their entireties. In some embodiments, the generated ligation products are amplified using one or both of inverse PCR and rolling circle amplification.

### EXAMPLE

### Background and Purpose

For applications in which high coverage of specific regions of a genome are required, target enrichment methods are used to increase signal of these targets. Deoxyribose Nucleic Acid (DNA) hybridization is a common technique to achieve such target enrichment. In brief, this technique uses custom DNA probes that select for the region(s) on interest and allow subsequent removal of off-target regions that do not bind to these probes (called a panel).

There are several essential components for a successful DNA hybridization reaction; one of which is a DNA denaturant. DNA denaturants are chemicals that ease the separation of double-stranded DNA into single stranded molecules. This is helpful in ensuring that only precise matches between the panel and a template are bound and miss-matches are not bound to the panel.

Pure dimethyl sulfoxide (DMSO) is a very useful DNA denaturant; all other things being equal, more DMSO in a hybridization reaction typically causes stands with more favorable complementarity to maintain the DNA duplex (sequence complementarity, GC-content and length all play a role too). Because of this behavior, DMSO is a common reagent in some DNA Hybrid capture assays.

DMSO, in its pure form, freezes at about 19°C (66°F) meaning it is at risk of freezing close to ambient temperatures. This poses a risk for automated liquid handling robotic systems, which often do not actively control the temperature of reagents like DMSO or other DNA denaturants. Often times automation solutions require the user to ensure the reagent is a liquid prior to use. This not only poses a risk from user error, but also makes full walk-away automation a challenge.

This experiment tested the performance of pure DMSO, and alternative reagents for use on automated platforms as a DNA denaturant. These three formulations are referred to herein as Hybridization Buffer 2 when discussed as a group of reagents that function in the same manner in this application.

### Design

This experiment was divided into six conditions (n=8 replicates per condition) to test our Hybridization Buffer 2 formulations and the effective amount of pure DMSO that is in each reaction. The conditions are listed below (Table 3).

**Table 3: Describes the six different conditions evaluated during this experiment including the overall or equivalent amount of pure DMSO in the hybridization reaction (column 2) and hybridization buffer 2 used (column 3).**

| **Count** | **Effective total DMSO in reaction (v/v %)** | **DMSO or equivalent used (Hybridization buffer 2)** | **Comment** |
|---|---|---|---|
| 1 | 15 | Pure DMSO (10% DMSO) | Control |
| 2 | 15 | 90%DMSO-10%water (90% DMSO) | Test formulation |
| 3 | 15 | Formulation Example 1 (50% DMSO) (see Table 1, herein) | Test formulation |
| 4 | 24.8 | Pure DMSO (10% DMSO) | Control |
| 5 | 24.8 | 90%DMSO-10%water (90% DMSO) | Test formulation |
| 6 | 24.8 | Formulation Example 1 (50% DMSO) (see Table 1, herein) | Test formulation |

Alternative DMSO formulations were selected to ensure the new DMSO replacement reagent would pose the lowest risk to current and future DNA hybridization workflows. Criteria included:
o Remain a liquid at or below about 13°C
o Should not introduce any new reagents into the hybridization reaction
o Pose no stability risk at storage temperatures of about -15 to about -20°C
o Perform the same as pure DMSO when added properly (control)

In this experiment the two pure DMSO alternatives used were identified as "90%DMSO-10%water" which described the formulation directly as the v/v percent of the two components. This DMSO mixture is a common alternative to pure DMSO especially in cold environments. Formulation Example 1 (described herein and as set forth in Table 1) was selected as it more readily mimics a previously optimized formulation for hybridization.

### Methods

### Nucleic acid preparation

Cell line DNA (NA12878 from Corriell Inst.) was buffer exchanged using HyperPure^{®} beads (Roche) and eluted in PCR-grade water. The eluted product was then normalized to about 50 ng input (in about 30.5 µL).

### Library preparation and target enrichment

Samples were processed using reagents and modified workflow steps from the KAPA NGHC products/workflow v3.0 (Roche) . Dual indexed libraries were normalized to 1 µg of DNA in 30 µL reactions in preparation for hybridization. Hybridization set-up generally followed the same KAPA NGHC workflow with the following key modifications:
o Reactions were not multiplexed before or during hybridization (but can be, if desired);
o Use of the DMSO or equivalent reagents instead of hybridization component H;
o Hybridization master mixes were created as follows (see Table 4);
o Panel (Twist Biosystems);
o Used custom oncology panel 314 Kb;
o Added 5 µL per reaction (0.23 fmol/probe/4µL reaction)

Reactions were then allowed to hybridize for about 16 hours at about 55°C and processed though target enrichment and final library amplification, again using the KAP NGHC workflow and reagents.

Samples were diluted to 4 nM and pooled with equal volumes. For this application, up 24 reactions were pooled in preparation for sequencing.

### Sequencing and analysis

Samples were sequenced on a NextSeq550/500 instrument using the High-output 300 cycle kits (illumine Inc.). BCL files were converted to fastq format and de-multiplexed. All reactions were sub-sampled to 2*10+07 total reads. Raw files were fed into an internal pipeline for germline analysis to generate sequencing QC metrics.

**Table 4: Illustrates the exact volumes for all hybridization mix components for a single reaction for all conditions tested (no overage added).**

| **Count** | **Effective total DMSO in reaction (v/v %)** | **DMSO or equivalent used (Hybridization buffer 2)** | **DMSO or equivalent component volume (µL)** | **Hybridization buffer 1 (µL)** | **Total volume (µL)** |
|---|---|---|---|---|---|
| 1 | 15 | Pure DMSO (100% DMSO) | 9.1 | 32.9 | 42 |
| 2 | 15 | 90%DMSO-10%water (90% DMSO) | 10.1 | 31.9 | 42 |
| 3 | 15 | Example formulation 1 (50% DMSO) | 18.1 | 23.9 | 42 |
| 4 | 24.8 | Pure DMSO (10% DMSO) | 15 | 27 | 42 |
| 5 | 24.8 | 90%DMSO-10%water (90% DMSO) | 16.6 | 25.4 | 42 |
| 6 | 24.8 | Example formulation 1 (50% DMSO) | 30 | 12 | 42 |

### Results

Performance evaluation of these Hybridization Buffer 2 formulations focused on hybridization efficiency and uniformity of the capture. Gross Hybridization efficiency is typically measured by the fraction of reads that cover the target area as a portion of all reads mapped to the genome of interest. This metric is often called on-target rate or on-target percentage. For this metric, higher values indicate an overall more efficient hybridization reaction. In the present experiment, on-target rates increased directly with DMSO content in the hybridization reaction. As this experiment manipulated the primary denaturant in the hybridization reaction, this metric was essential for performance monitoring. In this experiment, the higher Hybridization Buffer 2 conditions (24.8%) showed an about 15 to about 20 percentage point increase over the reactions with lower Hybridization Buffer 2 components (15%). Furthermore, within when the Hybridization Buffer 2 percentage was fixed (either 24.8 or 15%), there was little to no discernable capture performance difference with different formulations (see FIG. 1).

Though increasing DMSO content in a hybridization reaction can improve the overall efficiency, it is also critical that all targets are being sequenced at sufficient depth. Another important class of metrics for DNA hybridization is capture uniformity. There are many different ways to approach this (examples include: Percent of reads within +/- 25% from median depth of coverage, coverage by GC content, etc.). Here, Fold-80 base penalty was used because the pipeline tools used to calculate this metric are freely available (Broad Institute) (see FIG. 2). Fold-80 base penalty is a unit-less value indicating the number of (or fold) additional reads needed to get 80% of all target areas, at or above the current mean depth of coverage (applies to non-zero coverage areas only). A lower number is better and a value of 1 indicates the run is perfectly uniform. In this dataset, uniformity appeared to be well controlled regardless of the condition chosen, though the 24.8% Hybridization Buffer 2 had slightly better uniformity.

Fold-80 base penalty is a single number which means it is a quick and simple way to assess uniformity; however, some sub-optimal behavior can be hidden by using such a simple representation of that variable. To supplement the fold-80 base penalty metric, the uniformity of these hybridization conditions was examined based on the GC content coverage (%). All panel targets were measured based on the GC-content across all contiguous target areas (in 5% bins used). By measuring normalized coverage within these bins, the behavior of all targets with a given GC content can be quantified. A single line of best fit was used to explain this behavior for all hybridization component conditions; for reference a dotted line a 1 was added to show were the average should be (ideally). Finally, the total amount of targets in a given GC bin is also provided, in order to better inform how many targets may be effected by differing coverage in the plot. Looking at normalized GC coverage, the previous uniformity findings were able to be corroborated. Adding more Hybridization Buffer 2 appeared to increase the coverage of targets with high GC-content which offsets the lower coverage seen in all conditions for low GC-content targets. This as wis most prominent starting at about the 55% GC bin. Seeing as the number of target at or above that GC content was relatively low (accounting for about 20% of all targets) this lead to only a modest impact on overall uniformity (see FIGS. 3A and 3B).

By examining these target enrichment metrics, it has been shown that the two alternative Hybridization Buffer 2 formulations performed at least as well as the control (pure DMSO). This was true across all capture metrics, and within the range of hybridization mix formulations expected for this technique. In conclusion, this example illustrates that the two Hybridization Buffer 2 formulations retained the characteristics of pure DMSO, with added benefit of decreasing the melting point of the reagent by >> 10°C (data not shown). This information can be used to guide automated development of similar workflows and dramatically improve walk-away capabilities in this space.

## Claims

1. A hybridization buffer formulation comprising or consisting essentially of: (i) between about 36% to about 50% of DMSO by total volume of the hybridization buffer formulation; (ii) between about 0.0008% to about 0.0016% of one or more surfactants by total volume of the hybridization buffer formulation; (iii) between about 20% about 26% of one or more quaternary ammonium salts by total volume of the hybridization buffer formulation; (iv) between about 5% about 6.5% of one or more buffers by total volume of the hybridization buffer formulation; and (v) between 25% about 32% of one or more secondary salts by total volume of the hybridization buffer formulation,
wherein the one or more secondary salts are selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof, and
wherein the hybridization buffer formulation is free of formamide.

2. The hybridization buffer formulation of claim 1, wherein the one or more surfactants are anionic surfactants.

3. The hybridization buffer formulation of claim 1, wherein the one or more surfactants are selected from the group consisting of polysorbate 20, polysorbate 40, polysorbate 60, polysorbate 80, and combinations thereof.

4. The hybridization buffer formulation of claim 1, wherein the one or more surfactants comprises polysorbate 20.

5. The hybridization buffer formulation of claim 1, wherein the one or more quaternary ammonium salts are selected from the group consisting of tetramethylammonium chloride, tetraethylammonium chloride, tetrabutylammonium chloride, trimethylammonium chloride, Tetra-n-butylammonium chloride, and combinations thereof.

6. The hybridization buffer formulation of claim 1, wherein the one or more buffers are selected from the group consisting of 2-morpholinoethanesulfonic acid, 3-(N-morpholino)propanesulfonic acid, succinate, cacodylate, 4-(2-hydroxyethyl)-1-piperazineethanesulfonic acid, and combinations thereof.

7. The hybridization buffer formulation of claim 1, wherein the one or more surfactants is polysorbate 20, the one or more quaternary ammonium salts is tetramethylammonium chloride, the one or more buffers is 2-morpholinoethanesulfonic acid, and wherein the one or more secondary salts is N,N,N-trimethylglycine.

8. The hybridization buffer formulation of claim 1, wherein the hybridization buffer formulation comprises: (i) between about 48% to about 52% of the DMSO by total volume of the hybridization buffer formulation; (ii) between about 0.0008% to about 0.0016% of the one or more surfactants by total volume of the hybridization buffer formulation; (iii) between about 18% about 22% of the one or more quaternary ammonium salts by total volume of the hybridization buffer formulation; (iv) between about 4% about 6% of the one or more buffers by total volume of the hybridization buffer formulation; and (v) between 24% about 26% of the one or more secondary salts by total volume of the hybridization buffer formulation.

9. The hybridization buffer of claim 1, wherein the hybridization buffer formulation comprises: (i) between about 38% to about 42% of the DMSO by total volume of the hybridization buffer formulation; (ii) between about 0.0008% to about 0.0016% of the one or more surfactants by total volume of the hybridization buffer formulation; (iii) between about 22% about 26% of the one or more quaternary ammonium salts by total volume of the hybridization buffer formulation; (iv) between about 5% about 7% of the one or more buffers by total volume of the hybridization buffer formulation; and (v) between 28% about 31% of the one or more secondary salts by total volume of the hybridization buffer formulation.

10. A reaction mixture comprising: (i) between about 18% to about 38% DMSO by total volume of the reaction mixture; (ii) between about 0.0008% to about 0.002% of one or more surfactants by total volume of the reaction mixture; (iii) between about 22% about 33% of one or more quaternary ammonium salts by total volume of the reaction mixture; (iv) between about 5% about 8% of one or more buffers by total volume of the reaction mixture; (v) between 28% about 42% of one or more secondary salts by total volume of the reaction mixture; and (vi) between about 0% to about 0.04% water,
wherein the one or more secondary salts are selected from the group consisting of N,N,N-trimethylglycine, tetramethylammonium chloride, tetramethylammonium bromide, tetramethylammonium iodide, N,N,N-trimethylmethionine, N,N,N-trimethylisolucine, N,N,N-trimethylvaline, N,N,N-trimethylalanine, and combinations thereof, and
wherein the reaction mixture is free of formamide.

11. The reaction mixture of claim 10, wherein the one or more surfactants are selected from the group consisting of polysorbate 20, polysorbate 40, polysorbate 60, polysorbate 80, and combinations thereof.

12. The reaction mixture of claim 10, wherein the one or more quaternary ammonium salts are selected from the group consisting of tetramethylammonium chloride, tetraethylammonium chloride, tetrabutylammonium chloride, trimethylammonium chloride, Tetra-n-butylammonium chloride, and combinations thereof.

13. The reaction mixture of claim 10, wherein the one or more buffers are selected from the group consisting of 2-morpholinoethanesulfonic acid, 3-(N-morpholino)propanesulfonic acid, succinate, cacodylate, 4-(2-hydroxyethyl)-1-piperazineethanesulfonic acid, and combinations thereof.

14. A master mix comprising between about 62% and about 78% of the reaction mixture of any one of claims 10 to 13, with the remainder of the master mix comprising oligonucleotides or mixtures of oligonucleotides, wherein the master mix is free of formamide.

15. Use of the hybridization buffers of any one of claims 1-10 or reaction mixtures of any one of claims 11-13 in enriching a sample comprising one or more target nucleic acid sequences.

## Patentansprüche

1. Hybridisierungspufferformulierung, umfassend oder im Wesentlichen bestehend aus: (i) zwischen etwa 36% bis etwa 50% DMSO, bezogen auf das Gesamtvolumen der Hybridisierungspufferformulierung; (ii) zwischen etwa 0,0008% bis etwa 0,0016% eines oder mehrerer Tenside, bezogen auf das Gesamtvolumen der Hybridisierungspufferformulierung; (iii) zwischen etwa 20% und etwa 26% eines oder mehrerer quaternärer Ammoniumsalze, bezogen auf das Gesamtvolumen der Hybridisierungspufferformulierung; (iv) zwischen etwa 5% und etwa 6,5% eines oder mehrerer Puffer, bezogen auf das Gesamtvolumen der Hybridisierungspufferformulierung; und (v) zwischen 25% und etwa 32% eines oder mehrerer sekundärer Salze, bezogen auf das Gesamtvolumen der Hybridisierungspufferformulierung,
wobei das eine oder die mehreren sekundären Salze ausgewählt sind aus der Gruppe, bestehend aus N,N,N-Trimethylglycin, Tetramethylammoniumchlorid, Tetramethylammoniumbromid, Tetramethylammoniumiodid, N,N,N-Trimethylmethionin, N,N,N-Trimethylisoluzin, N,N,N-Trimethylvalin, N,N,N-Trimethylalanin und Kombinationen davon, und
wobei die Hybridisierungspufferformulierung frei von Formamid ist.

2. Hybridisierungspufferformulierung nach Anspruch 1, wobei das eine oder die mehreren Tenside anionische Tenside sind.

3. Hybridisierungspufferformulierung nach Anspruch 1, wobei das eine oder die mehreren Tenside ausgewählt sind aus der Gruppe, bestehend aus Polysorbat 20, Polysorbat 40, Polysorbat 60, Polysorbat 80 und Kombinationen davon.

4. Hybridisierungspufferformulierung nach Anspruch 1, wobei das eine oder die mehreren Tenside Polysorbat 20 umfassen.

5. Hybridisierungspufferformulierung nach Anspruch 1, wobei das eine oder die mehreren quaternären Ammoniumsalze ausgewählt sind aus der Gruppe, bestehend aus Tetramethylammoniumchlorid, Tetraethylammoniumchlorid, Tetrabutylammoniumchlorid, Trimethylammoniumchlorid, Tetra-n-butylammoniumchlorid und Kombinationen davon.

6. Hybridisierungspufferformulierung nach Anspruch 1, wobei der eine oder die mehreren Puffer ausgewählt sind aus der Gruppe, bestehend aus 2-Morpholinoethansulfonsäure, 3-(N-Morpholino)propansulfonsäure, Succinat, Cacodylat, 4-(2-Hydroxyethyl)-1-piperazinethansulfonsäure und Kombinationen davon.

7. Hybridisierungspufferformulierung nach Anspruch 1, wobei das eine oder die mehreren Tenside Polysorbat 20 sind, das eine oder die mehreren quaternären Ammoniumsalze Tetramethylammoniumchlorid sind, der eine oder die mehreren Puffer 2-Morpholinoethansulfonsäure sind und wobei das eine oder die mehreren sekundären Salze N,N,N-Trimethylglycin sind.

8. Hybridisierungspufferformulierung nach Anspruch 1, wobei die Hybridisierungspufferformulierung umfasst: (i) zwischen etwa 48% bis etwa 52% DMSO, bezogen auf das Gesamtvolumen der Hybridisierungspufferformulierung; (ii) zwischen etwa 0,0008% bis etwa 0,0016% des einen oder der mehreren Tenside, bezogen auf das Gesamtvolumen der Hybridisierungspufferformulierung; (iii) zwischen etwa 18% und etwa 22% des einen oder der mehreren quaternären Ammoniumsalze, bezogen auf das Gesamtvolumen der Hybridisierungspufferformulierung; (iv) zwischen etwa 4% und etwa 6% des einen oder der mehreren Puffer, bezogen auf das Gesamtvolumen der Hybridisierungspufferformulierung; und (v) zwischen 24% und etwa 26% des einen oder der mehreren sekundären Salze, bezogen auf das Gesamtvolumen der Hybridisierungspufferformulierung.

9. Hybridisierungspufferformulierung nach Anspruch 1, wobei die Hybridisierungspufferformulierung umfasst: (i) zwischen etwa 38% bis etwa 42% DMSO, bezogen auf das Gesamtvolumen der Hybridisierungspufferformulierung; (ii) zwischen etwa 0,0008% bis etwa 0,0016% des einen oder der mehreren Tenside, bezogen auf das Gesamtvolumen der Hybridisierungspufferformulierung; (iii) zwischen etwa 22% und etwa 26% des einen oder der mehreren quaternären Ammoniumsalze, bezogen auf das Gesamtvolumen der Hybridisierungspufferformulierung; (iv) zwischen etwa 5% und etwa 7% des einen oder der mehreren Puffer, bezogen auf das Gesamtvolumen der Hybridisierungspufferformulierung; und (v) zwischen 28% und etwa 31% des einen oder der mehreren sekundären Salze, bezogen auf das Gesamtvolumen der Hybridisierungspufferformulierung.

10. Reaktionsmischung, umfassend: (i) zwischen etwa 18% bis etwa 38% DMSO, bezogen auf das Gesamtvolumen der Reaktionsmischung; (ii) zwischen etwa 0,0008% bis etwa 0,002% eines oder mehrerer Tenside, bezogen auf das Gesamtvolumen der Reaktionsmischung; (iii) zwischen etwa 22% und etwa 33% eines oder mehrerer quaternärer Ammoniumsalze, bezogen auf das Gesamtvolumen der Reaktionsmischung; (iv) zwischen etwa 5% und etwa 8% eines oder mehrerer Puffer, bezogen auf das Gesamtvolumen der Reaktionsmischung; (v) zwischen 28% und etwa 42% eines oder mehrerer sekundärer Salze, bezogen auf das Gesamtvolumen der Reaktionsmischung; und (vi) zwischen etwa 0% und etwa 0,04% Wasser, wobei das eine oder die mehreren sekundären Salze ausgewählt sind aus der Gruppe, bestehend aus N,N,N-Trimethylglycin, Tetramethylammoniumchlorid, Tetramethylammoniumbromid, Tetramethylammoniumiodid, N,N,N-Trimethylmethionin, N,N,N-Trimethylisoluzin, N,N,N-Trimethylvalin, N,N,N-Trimethylalanin und Kombinationen davon, und wobei die Reaktionsmischung frei von Formamid ist.

11. Reaktionsmischung nach Anspruch 10, wobei das eine oder die mehreren Tenside ausgewählt sind aus der Gruppe, bestehend aus Polysorbat 20, Polysorbat 40, Polysorbat 60, Polysorbat 80 und Kombinationen davon.

12. Reaktionsmischung nach Anspruch 10, wobei das eine oder die mehreren quaternären Ammoniumsalze ausgewählt sind aus der Gruppe, bestehend aus Tetramethylammoniumchlorid, Tetraethylammoniumchlorid, Tetrabutylammoniumchlorid, Trimethylammoniumchlorid, Tetra-n-butylammoniumchlorid und Kombinationen davon.

13. Reaktionsmischung nach Anspruch 10, wobei der eine oder die mehreren Puffer ausgewählt sind aus der Gruppe, bestehend aus 2-Morpholinoethansulfonsäure, 3-(N-Morpholino)propansulfonsäure, Succinat, Cacodylat, 4-(2-Hydroxyethyl)-1-piperazinethansulfonsäure und Kombinationen davon.

14. Mastermischung, umfassend zwischen etwa 62% und etwa 78% der Reaktionsmischung nach einem der Ansprüche 10 bis 13, wobei der Rest der Mastermischung Oligonukleotide oder Mischungen von Oligonukleotiden umfasst, wobei die Mastermischung frei von Formamid ist.

15. Verwendung der Hybridisierungspuffer nach einem der Ansprüche 1-10 oder der Reaktionsmischungen nach einem der Ansprüche 11-13 beim Anreichern einer Probe, die eine oder mehrere Zielnukleinsäuresequenzen umfasst.

## Revendications

1. Formulation de tampons d'hybridation comprenant ou essentiellement constituée de : (i) entre environ 36 % et environ 50 % de DMSO par volume total de la formulation de tampons d'hybridation ; (ii) entre environ 0,0008 % et environ 0,0016 % d'un ou de plusieurs tensioactifs par volume total de la formulation de tampons d'hybridation ; (iii) entre environ 20 % et environ 26 % d'un ou de plusieurs sels d'ammonium quaternaire par volume total de la formulation de tampons d'hybridation ; (iv) entre environ 5 % et environ 6,5 % d'un ou de plusieurs tampons par volume total de la formulation de tampons d'hybridation ; et (v) entre 25 % et environ 32 % d'un ou de plusieurs sels secondaires par volume total de la formulation de tampons d'hybridation,
dans laquelle le ou les sels secondaires sont choisis dans le groupe constitué par la N,N,N-triméthylglycine, le chlorure de tétraméthylammonium, le bromure de tétraméthylammonium, l'iodure de tétraméthylammonium, la N,N,N-triméthylméthionine, la N,N,N-triméthylisolucine, la N,N,N-triméthylvaline, la N,N,N-triméthylalanine et les combinaisons de ceux-ci, et
dans laquelle la formulation de tampons d'hybridation est exempte de formamide.

2. Formulation de tampons d'hybridation selon la revendication 1, dans laquelle le ou les tensioactifs sont des tensioactifs anioniques.

3. Formulation de tampons d'hybridation selon la revendication 1, dans laquelle le ou les tensioactifs sont choisis dans le groupe constitué par le polysorbate 20, le polysorbate 40, le polysorbate 60, le polysorbate 80 et des combinaisons de ceux-ci.

4. Formulation de tampons d'hybridation selon la revendication 1, dans laquelle le ou les tensioactifs comprennent le polysorbate 20.

5. Formulation de tampons d'hybridation selon la revendication 1, dans laquelle le ou les sels d'ammonium quaternaire sont choisis dans le groupe constitué par le chlorure de tétraméthylammonium, le chlorure de tétraéthylammonium, le chlorure de tétrabutylammonium, le chlorure de triméthylammonium, le chlorure de tétra-n-butylammonium et des combinaisons de ceux-ci.

6. Formulation de tampons d'hybridation selon la revendication 1, dans laquelle le ou les tampons sont choisis dans le groupe constitué par l'acide 2-morpholinoéthanesulfonique, l'acide 3-(N-morpholino)propanesulfonique, le succinate, le cacodylate, l'acide 4-(2-hydroxyéthyl)-1-pipérazineéthanesulfonique et des combinaisons de ceux-ci.

7. Formulation de tampons d'hybridation selon la revendication 1, dans laquelle le ou les tensioactifs sont le polysorbate 20, le ou les sels d'ammonium quaternaire sont le chlorure de tétraméthylammonium, le ou les tampons sont l'acide 2-morpholinoéthanesulfonique, et dans laquelle le ou les sels secondaires sont la N,N,N-triméthylglycine.

8. Formulation de tampons d'hybridation selon la revendication 1, dans laquelle la formulation de tampons d'hybridation comprend : (i) entre environ 48 % et environ 52 % du DMSO par volume total de la formulation de tampons d'hybridation ; (ii) entre environ 0,0008 % et environ 0,0016 % du ou des tensioactifs par volume total de la formulation de tampons d'hybridation ; (iii) entre environ 18 % et environ 22 % du ou des sels d'ammonium quaternaire par volume total de la formulation de tampons d'hybridation ; (iv) entre environ 4 % et environ 6 % du ou des tampons par volume total de la formulation de tampons d'hybridation ; et (v) entre 24 % et environ 26 % du ou des sels secondaires par volume total de la formulation de tampons d'hybridation.

9. Tampon d'hybridation selon la revendication 1, dans lequel la formulation de tampons d'hybridation comprend : (i) entre environ 38 % et environ 42 % du DMSO par volume total de la formulation de tampons d'hybridation ; (ii) entre environ 0,0008 % et environ 0,0016 % du ou des tensioactifs par volume total de la formulation de tampons d'hybridation ; (iii) entre environ 22 % et environ 26 % du ou des sels d'ammonium quaternaire par volume total de la formulation de tampons d'hybridation ; (iv) entre environ 5 % et environ 7 % du ou des tampons par volume total de la formulation de tampons d'hybridation ; et (v) entre 28 % et environ 31 % du ou des sels secondaires par volume total de la formulation de tampons d'hybridation.

10. Mélange réactionnel comprenant : (i) entre environ 18 % et environ 38 % de DMSO par volume total du mélange réactionnel ; (ii) entre environ 0,0008 % et environ 0,002 % d'un ou de plusieurs tensioactifs par volume total du mélange réactionnel ; (iii) entre environ 22 % et environ 33 % d'un ou de plusieurs sels d'ammonium quaternaire par volume total du mélange réactionnel ; (iv) entre environ 5 % et environ 8 % d'un ou de plusieurs tampons par volume total du mélange réactionnel ; (v) entre 28 % et environ 42 % d'un ou de plusieurs sels secondaires par volume total du mélange réactionnel ; et (vi) entre environ 0 % et environ 0,04 % d'eau,
dans lequel le ou les sels secondaires sont choisis dans le groupe constitué par la N,N,N-triméthylglycine, le chlorure de tétraméthylammonium, le bromure de tétraméthylammonium, l'iodure de tétraméthylammonium, la N,N,N-triméthylméthionine, la N,N,N-triméthylisolucine, la N,N,N-triméthylvaline, la N,N,N-triméthylalanine et des combinaisons de ceux-ci, et
dans lequel le mélange réactionnel est exempt de formamide.

11. Mélange réactionnel selon la revendication 10, dans lequel le ou les tensioactifs sont choisis dans le groupe constitué par le polysorbate 20, le polysorbate 40, le polysorbate 60, le polysorbate 80 et des combinaisons de ceux-ci.

12. Mélange réactionnel selon la revendication 10, dans lequel le ou les sels d'ammonium quaternaire sont choisis dans le groupe constitué par le chlorure de tétraméthylammonium, le chlorure de tétraéthylammonium, le chlorure de tétrabutylammonium, le chlorure de triméthylammonium, le chlorure de tétra-n-butylammonium et des combinaisons de ceux-ci.

13. Mélange réactionnel selon la revendication 10, dans lequel le ou les tampons sont choisis dans le groupe constitué par l'acide 2-morpholinoéthanesulfonique, l'acide 3-(N-morpholino)propanesulfonique, le succinate, le cacodylate, l'acide 4-(2-hydroxyéthyl)-1-pipérazineéthanesulfonique et des combinaisons de ceux-ci.

14. Mélange maître comprenant entre environ 62 % et environ 78 % du mélange réactionnel selon l'une quelconque des revendications 10 à 13, le reste du mélange maître comprenant des oligonucléotides ou des mélanges d'oligonucléotides, dans lequel le mélange maître est exempt de formamide.

15. Utilisation des tampons d'hybridation selon l'une quelconque des revendications 1 à 10 ou des mélanges réactionnels selon l'une quelconque des revendications 11 à 13 dans l'enrichissement d'un échantillon comprenant une ou plusieurs séquences d'acides nucléiques cibles.
